# EUROPEAN PATENT SPECIFICATION

(11) **EP 1 732 950 B1**
(45) Date of publication and mention of the grant of the patent: **18.05.2011**
(21) Application number: 05734321.2
(22) Date of filing: 08.04.2005
(51) Int. Cl.: C07K 16/28, A61K 39/395, C12N 15/13, C12N 15/63, C12N 5/10

(54) **ANTIBODIES TO ERYTHROPOIETIN RECEPTOR AND USES THEREOF**
ANTIKÖRPER GEGEN DEN ERYTHROPOIETINREZEPTOR UND VERWENDUNGEN DAVON
ANTICORPS DIRIGES CONTRE LE RECEPTEUR DE L'ERYTHROPOIETINE ET UTILISATIONS ASSOCIEES

(30) Priority: 09.04.2004 US 821497; 12.04.2004 US 822306
(43) Date of publication of application: 20.12.2006
(60) Divisional application: 08165634.0
(73) Proprietor: ABBOTT LABORATORIES, Abbott Park IL 60064-3500 (US)
(72) Inventor: REILLY, Edward, B., Libertyville, Illinois 60048 (US); LACY, Susan, E., Shrewsbury, Massachusetts 01545 (US); FUNG, Emma, Shrewsbury, Massachusetts 01545 (US); BELK, Jonathan, P., Sterling, Massachusetts 01564 (US); ROGUSKA, Michael, Ashland, MA 01721 (US)
(74) Representative: Modiano, Micaela Nadia
(86) International application number: PCT/US2005/011956
(87) International publication number: WO 2005/100403

(56) References cited:
- WO-A-96/40231
- WO-A-97/48729
- WO-A-99/55369
- WO-A-2004/035603
- MAYNARD JA ET AL: NATURE BIOTECHNOL, vol. 20, 2002, pages 597-601,
- JOHNSON S ET AL: J INFECT DIS, vol. 180, 1999, pages 35-40,
- BARBAS CF ET AL: PROC NATL ACAD SCI, vol. 91, 1994, pages 3809-3813,
- WU H ET AL: PROC NATL ACAD SCI, vol. 95, 1998, pages 6037-6042,
- ADAMS GP ET AL: CANCER RES, vol. 58, 1998, pages 485-490,
- VITI F ET AL: CANCER RES, vol. 59, 1999, pages 347-352,
- WARK KL ET AL: ADV DRUG DELIVERY REVIEWS, vol. 58, 2006, pages 658-669,

## Description

### BACKGROUND OF THE INVENTION

Erythropoietin ("Epo") is a glycoprotein that is the primary regulator of erythropoiesis. Specifically, Epo is responsible for promoting the growth, differentiation and survival of erythroid progenitors, which give rise to mature red blood cells. In response to changes in the level of oxygen in the blood and tissues, erythropoietin appears to stimulate both proliferation and differentiation of immature erythroblasts. It also functions as a growth factor, stimulating the mitotic activity of erythroid progenitor cells, such as erythrocyte burst forming and colony-forming units. It also acts as a differentiation factor, triggering transformation of an erythrocyte colony-forming-unit into a proerythroblast (See Erslev, A., New Eng. J. Med., 316:101-103 (1987)).

Epo has a molecular weight of about 34,000 daltons and can occur in three forms - alpha, beta and asialo. During mid- to late gestation, Epo is synthesized in the fetal liver. Subsequently, Epo is synthesized in the kidney, circulates in the plasma and is excreted in the urine.

Human urinary Epo has been isolated and purified (See, Miyake et al., J. Biol. Chem., 252:5558 (1977)). Moreover, methods for identifying, cloning and expressing genes encoding Epo (See U.S. Patent 4,703,008) as well as purifying recombinant Epo from a cell medium (See U.S. Patent 4,667,016) are known in the art.

The activity of Epo is mediated through the binding and activation of a cell surface receptor referred to as the erythropoietin receptor (EpoR). The Epo receptor belongs to the cytokine receptor superfamily and is believed to contain at least two distinct polypeptides, a 55-72 kDa species and a 85-100 kDa species (See U.S. Patent 6,319,499, Mayeux et al., J. Biol. Chem, 266:23380 (1991), McCaffery et al., J. Biol. 6,319,499, Mayeux et aL, J. Biol. Chem, 266:23380 (1991), McCaffery et al., J. Biol. Chem., 264:10507 (1991)). Other studies have revealed other polypeptide complexes of Epo receptor having molecular weights such as 110, 130 and 145 kDa (See U.S. Patent 6,319,499).

Both the murine and human Epo receptors have been cloned and expressed (See D'Andrea et al., Cell, 57:277 (1989); Jones et aL, Blood, 76:31 (1990); Winkelmarm et al., Blood, 76:24 (1990); WO 90/08822/ U.S. Patent 5,278,065). The full length human Epo receptor is a 483 amino acid transmembrane protein with an approximately 25 amino acid signal peptide (See U.S. Patent 6,319,499). The human receptor demonstrates about 82% amino acid sequence homology with the murine receptor. *Id*.

In the absence of ligand the Epo receptor exists in a preformed dimer. The binding of Epo to its receptor causes a conformational change such that the cytoplasmic domains are placed in close proximity. While not completely understood, it is believed that this "dimerization" plays a role in the activation of the receptor. The activation of the Epo receptor results in a number of biological effects. Some of these activities include stimulation of proliferation, stimulation of differentiation and inhibition of apoptosis (See U.S. Patent 6,319,499, Liboi et al,. PNAS USA, 90:11351 (1993), Koury, Science, 248:378 (1990)).

It is the relationship between the Epo receptor dimerization and activation that can be used to identify compounds (i.e. such as antibodies) other than Epo that are capable of: (1) dimerizing the Epo receptor; and (2) activating the receptor. These compounds would be useful in treating mammals suffering from anemia and in identifying mammals having a dysfunctional Epo receptor.

In WO 96/40231 it is describe a method for generating antibodies which are receptor agonists. For example, it is described an Epo agonist antibody which may be used in the treatment of anemia.

WO 99/55369 describes antibodies having reduced immunogenicity in humans and methods for their preparation comprising CDR-grafting from donor antibodies of a non-human species. For example, the preparation of an anti-Epo receptor antibody by CDR-grafting from a murine donor antibody to a non-human primate acceptor antibody is described.

In WO 97/048729 it is described an antibody which activates Epo receptors and is useful for treating disorders related to activation of the Epo receptor. For example, the MAb34 antibody is described which is capable of binding the Epo receptor and exhibits a K*_{off}* of 1.7x10⁻²s⁻¹.

WO 04/035603 relates to antibodies and antibody fragments that bind to and activate an Epo receptor, such as the Ab 198 antibody having a K_{d} of 13.9 nM.

### SUMMARY OF THE INVENTION

The invention provides an isolated antibody, or an antigen-binding portion thereof, that dissociates from human erythropoietin receptor (EpoR) with a K*_{off}* rate constant of greater than about 1.3x10⁻³s⁻¹ and that activates an endogenous activity of said human EpoR in a mammal, said antibody or antigen-binding portion thereof comprising a heavy chain variable region (HCVR) and a light chain variable region, wherein the heavy chain variable region comprises an amino acid sequence of Formula I: wherein X₁ is independently selected from the group consisting of tyrosine (Y), glycine (G) and alanine (A); X₂ is independently selected from the group consisting of tyrosine (Y), glycine (G), alanine (A), glutamine (E) and aspartic acid (D); and X₃ is independently selected from the group consisting of serine (S), glycine (G), glutamine (E) and threonine (T) with the proviso that X1-X2-X3 is other than Y-Y-S, and the light chain variable region comprises an amino acid sequence of SEQ ID NO:17. The antibodies of the invention are characterized by binding to EpoR with low affinity and dissociating from human erythropoietin receptor (EpoR) with a fast off-rate. The antibodies or antigen-binding portion thereof can be full-length (e.g. an IgG2) or can comprise only an antigen-binding portion (e. g. an F(ab')₂). In a preferred embodiment, the antibodies of the invention bind to EpoR with a K*_{d}* of about 7 nM or greater. In a more preferred embodiment, the antibody or antigen-binding portion thereof binds to EpoR with a K*_{d}* of about 8.5 nM or greater. In an even more preferred embodiment, the antibody or antigen-binding portion thereof binds to EpoR with a K*_{d}* of about 20 nM and most preferably, with a K*_{d}* of about 32 nM.

In a more preferred embodiment, the antibody or antigen-binding portion thereof dissociates from EpoR with an K*_{off}* rate constant selected from the group consiting of about 1.4 x 10⁻³ s⁻¹ or greater, about 1.9 x 10⁻³ s⁻¹ and about 4.8 x 10⁻³ s⁻¹ as determined by surface plasmon resonance.

The antibody or antigen-binding portion thereof may be a monoclonal antibody. In a preferred embodiment, the antibody or antigen-binding portion thereof is an IgG2 isotype. In a further embodiment the antibody or antigen-binding portion thereof is a human antibody.

In another embodiment, the antibody or antigen-binding portion thereof has a heavy chain variable region (HCVR) having an amino acid sequence selected from the group consisting SEQ ID NO: 7, SEQ ID NO: 8, SEQ ID NO: 9, SEQ ID NO: 10, SEQ ID NO: 11, SEQ ID NO: 12, SEQ ID NO: 13 and SEQ ID NO: 14.

Another aspect of the invention pertains to the antibody or antigen-binding portion thereof of the present disclosure for activating an endogenous activity of a human erythropoietin receptor for treating anemia, hypoxemia, chronic tissue hypoxia, heart failure, ischemic heart disease, renal failure, neural cell damage or tissue damage, or for promoting wound healing in a mammal, by administration to the mammal of a therapeutically effective amount of the antibody or antigen-binding portion thereof.

In another aspect, the invention pertains to the antibody or antigen-binding portion thereof of the present disclosure for treating a mammal suffering aplasia, by administration to a mammal in need of treatment of a therapeutically effective amount of the antibody or antigen-binding portion thereof.

In another aspect, the invention pertains to the antibody or antigen-binding portion thereof of the present disclosure for treating a mammal suffering anemia, by administration to a mammal in need of treatment of a therapeutically effective amount of the antibody or antigen-binding portion thereof.

In another aspect, the invention pertains to a pharmaceutical composition comprising a therapeutically effective amount of an antibody or antigen-binding portion thereof of the invention and a pharmaceutically acceptable excipient.

In another aspect, the invention pertains to an isolated or purified polynucleotide sequence which encodes a polypeptide comprising an amino acid sequence of Formula I: wherein X₁ is independently selected from the group consisting of tyrosine (Y), glycine (G) and alanine (A); X₂ is independently selected from the group consisting of tyrosine (Y), glycine (G), alanine (A), glutamine (E) and aspartic acid (D); and X₃ is independently selected from the group consisting of serine (S), glycine (G), glutamine (E) and threonine (T) with the proviso that X1-X2-X3 is other than Y-Y-S. In other preferred embodiments, the polynucleotide encodes a polypeptide selected from the group consisting of SEQ ID NO:7, SEQ ID NO:8, SEQ NO:9, SEQ ID NO:10, SEQ ID NO:11, SEQ ID NO:12, SEQ ID NO:13 and SEQ ID NO:14.

In another aspect the invention pertains to recombinant expression vectors comprising the polynucleotides set forth herein. The invention also pertains to a host cell comprising the recombinant expression vectors. Preferably, the host cell is a eucaryotic cell, mammalian cell, yeast cell or bacterial cell. More preferably, the host cell is a CHO cell, COS cell, or HEK-293 cell.

In another aspect, the invention pertains to polypeptide sequences encoded by said polynucleotide sequences set forth herein.

### BRIEF DESCRIPTION OF THE DRAWINGS

FIG. 1 represents a schematic drawing of an scFv construct, including tether and scFv linkers.

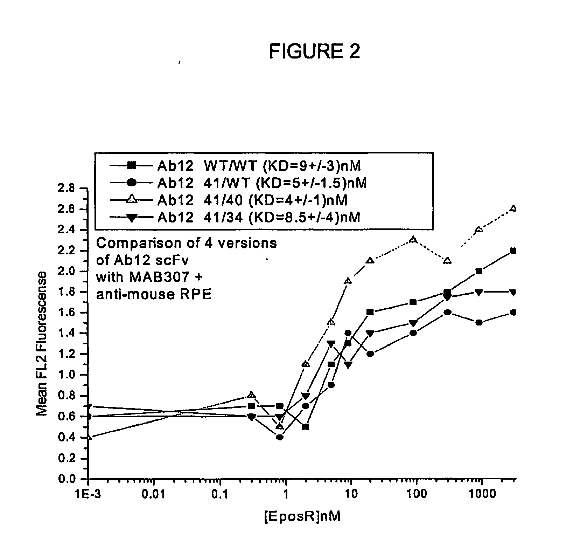

FIG. 2 is a graph showing equilibrium binding of soluble EpoR with various Ab12 scFv constructs expressed on the surface of yeast cells. In FIG. 2, -■- represents an Ab12 scFv construct comprising a Gly/Ser linker (WT), GGGGSGGGGSGGGGS (SEQ ID NO:1) in both the tether and scFv linker positions, -•- represents an Ab12 scFv construct comprising linker 41, GENKVEYAPALMALS (SEQ ID NO:2) in the tether position and the Gly/Ser linker (WT) (SEQ ID NO:1) in the scFv linker position, -▲- represents an Ab12 scFv construct comprising linker 41 (SEQ ID NO:2) in the tether position and linker 40, GPAKELTPLKEAKVS (SEQ ID NO:3) in the scFv linker position, and -▼- represents an Ab12 scFv construct comprising linker 41 (SEQ ID NO:2) in the tether position and linker 34, GHEAAAVMQVQYPAS (SEQ ID NO:4) in the scFv linker position.
FIG. 3 represents an off-rate analysis of Ab12 41/40 scFv.
FIG. 4 shows a schematic representation of the construction method for generating CDR mutagenic libraries in yeast.
FIG. 5 shows a schematic representation of Ab12 scFv heavy chain CDR mutagenic libraries. Library names are indicated to the left of each 3 amino acid sequence subjected to randomization. The sequences of Ab12 CDRs are shown below each CDR.
FIG. 6 shows a schematic representation of Ab12 scFv light chain CDR mutagenic libraries. Library names are indicated to the left of each 3 amino acid sequence subjected to randomization. The sequences of Ab12 CDRs are shown below each CDR
FIG. 7 is a chart showing the amino acid sequences of the heavy chain variable regions of the germline from which Ab12.6 and Ab12.6 related antibodies were derived (SEQ ID NO:5), Ab12 (SEQ ID NO:6), Ab12.6 (SEQ ID NO:7), Ab12.56 (SEQ ID NO:8), Ab12.118 (SEQ ID NO:9), Ab12.119 (SEQ ID NO:10), Ab12.120 (SEQ ID NO:11), Ab12.121 (SEQ ID NO:12), Ab12.122 (SEQ ID NO:13), Ab 12.123 (SEQ ID NO:14) and a consensus sequence (SEQ ID NO:15).
FIG. 8 is a chart showing the amino acid sequences of the light chain variable regions of the germline (SEQ ID NO:16) from which Ab12.6 and Ab12.6 related antibodies were derived, Ab12 (SEQ ID NO:17), Ab12.6 (SEQ ID NO:17) and Ab12.56 (SEQ ID NO:17), Ab12.118 (SEQ ID NO:17), Ab12.119 (SEQ ID NO:17), Ab12.120 (SEQ ID NO:17), Ab12.121 (SEQ ID NO:17), Ab 12.122 (SEQ ID NO:17), Ab12.123 (SEQ ID NO:17).
FIG. 9(a)-(i) shows the nucleic acid sequences of the heavy chain variable regions of Ab12, Ab12.6 and Ab12.6-related antibodies. Single letter codes representing the amino acids encoded by the nucleic acid sequences are shown on top.
FIG. 10 shows the nucleic acid sequences of the light chain variable region of Ab12, Ab12.6 and Ab12.6-related antibodies. Single letter codes representing the amino acids encoded by the nucleic acid sequences are shown on top.
FIG. 11 shows a graph of EC₅₀ and Emax values of Ab12.6 and Ab12.6-related antibodies. EC₅₀ values represent the concentration of antibody or Epo at which 50% maximal cell proliferation is achieved (50% of the slope of a sigmoidal curve). The Emax values represent the maximum number of cells which this EC₅₀ produces (as measured by absorbance).
FIG. 12 is a graph showing the formation of CFU-E (colony forming unit-erythroid) from human bone marrow in response to treatment with Epogen, Ab12, Aranesp™, Ab12.6 and isotype control.
FIG. 13 is a graph showing the formation of CFU-E (colony forming unit-erythroid) from mEpoR-/-, hEpoR+ transgenic mice bone marrow derived cells in response to treatment with Epogen, Ab12, Aranesp™, Ab12.6 and isotype control.
FIG. 14 is a graph showing change in hematocrit in mEpoR-/-, hEpoR+ transgenic mice over 28 days after administration of a single dose of Ab12.6 on day 0 versus two doses of Aranesp™ administered on day 0 and day 14. In FIG. 14, -◆-represents no treatment, -■- represents isotype control, -▲- represents Aranesp™ (3 µg/kg, 2x), -x- represents Ab12 (0.8 mg/kg), and -|- represents Ab12.6 (0.8 mg/kg).
FIG. 15 is a computer generated scan of a Western blot showing that Ab12.6 interacts with recombinant EpoR extracellular domain only under native, and not under denaturing conditions, indicating that Ab 12.6 recognizes a conformational-dependent epitope.
FIG. 16 is a graph showing that the monomeric Fab fragment derived from Ab12.6 activates EpoR and stimulates the proliferation of the human F36e erythroleukemic cell line.

### DETAILED DESCRIPTION OF THE INVENTION

This invention pertains to isolated human antibodies, or antigen-binding portions thereof, that bind to human erythropoietin with low affinity, a fast off-rate and activation or agonistic activity to the EpoR. Various aspects of the invention relate to antibodies and antibody fragments, and pharmaceutical compositions thereof, as well as nucleic acids, recombinant expression vectors and host cells for making such antibodies and fragments. Use of the antibody or antigen-binding portion thereof of the present disclosure for activating an endogenous activity of a human erythropoietin receptor for treating anemia, hypoxemia, chronic tissue hypoxia, heart failure, ischemic heart disease, renal failure, neural cell damage or tissue damage, for promoting wound healing in a mammal, for treating aplasia, or for treating anemia, is also encompassed by the invention. Unless otherwise defined herein, scientific and technical terms used in connection with the present invention shall have the meanings that are commonly understood by those of ordinary skill in the art. Further, unless otherwise required by context, singular terms shall include pluralities and plural terms shall include the singular. In this application, the use of "or" means "and/or" unless stated otherwise. Furthermore, the use of the term "including", as well as other forms, such as "includes" and "included", is not limiting. Also, terms such as "element" or "component" encompass both elements and components comprising one unit and elements and components that comprise more than one subunit unless specifically stated otherwise.

Generally, nomenclatures used in connection with, and techniques of, cell and tissue culture, molecular biology, immunology, microbiology, genetics and protein and nucleic acid chemistry and hybridization described herein are those well known and commonly used in the art The methods and techniques of the present invention are generally performed according to conventional methods well known in the art and as described in various general and more specific references that are cited and discussed throughout the present specification unless otherwise indicated. Enzymatic reactions and purification techniques are performed according to manufacturer's specifications, as commonly accomplished in the art or as described herein. The nomenclatures used in connection with, and the laboratory procedures and techniques of, analytical chemistry, synthetic organic chemistry, and medicinal and pharmaceutical chemistry described herein are those well known and commonly used in the art. Standard techniques are used for chemical syntheses, chemical analyses, pharmaceutical preparation, formulation, and delivery, and treatment of patients.

In order that the present invention may be more easily understood, certain terms first are defined.

The term "antibody" (abbreviated herein as Ab), as used herein is intended to refer to immoglobulin molecules comprised of four polypeptide chains, two heavy (H) chains and two light (L) chains interconnected by disulfide bonds. Each heavy chain is composed of a heavy chain variable region (abbreviated herein as HCVR or VH) and a heavy chain constant region (abbreviated herein as CH). The heavy chain constant region is comprised of three domains, CH1, CH2 and CH3. Each light chain is comprised of a light chain variable region (abbreviated herein as LCVR or VL) and a light chain constant region. The light chain constant region is composed of one domain, CL. The VH and VL regions can be further subdivided into regions of hypervariability, termed complementarity determining regions (CDR), interspersed with region that are more conserved, termed framework regions (FR). Each VH and VL is composed of three CDRs and four FRs respectively, arranged from amino-terminus to carboxy-terminus in the following order. FR1, CDR1, FR2, CDR2, FR3, CDR3, FR4 (sometimes referred to as "I").

Furthermore, the term "antibody" is used in the broadest sense and specifically covers monoclonal antibodies (including full length monoclonal antibodies), polyclonal antibodies, multispecific antibodies (e.g. bispecific antibodies), and antibody fragments so long as they exhibit the desired biological activity.

The term "antigen-binding portion" of an Ab (or simply "antibody portion"), as used herein, refers to one or more fragments of an antibody that retain the ability to specifically bind to an antigen (e.g. human EpoR). It has been shown that the antigen-binding function of an Ab can be performed by fragments of a full-length Ab. Examples of binding fragments encompassed within the term "antigen-binding portion" of an Ab include (i) an Fab fragment, a monovalent fragment consisting of the VL, VH, CL and CHl domains, (ii) an F(ab')₂ fragment, a bivalent fragment comprising two Fab fragments linked by a disulfide bridge at the hinge region; (iii) an Fd fragment consisting of the VH and CH1 domains, (iv) an Fv fragment consisting of the VL and VH domains of a single arm of an Ab, (v) a dAb fragment (Ward et al., (1989) Nature 341:544-546), which consists of a VH domain; and (vi) an isolated CDR. Furthermore, although the two domains of the Fv fragment, VL and VH, are coded for by separate genes, they can be joined, using recombinant methods, by a synthetic linker that enables them to be made as a single protein chain in which the VL and VH regions pair to form monovalent molecules (known as single chain Fv (scFv); see e.g., Bird et al. (1988) Science 242:423-426; and Huston et al. (1988) Proc. Natl. Acad. Sci.*.* Such single chain Abs are intended to be encompassed within the term "antigen-binding portion" of an Ab. Other forms of single chain antibodies, such as diabodies are also encompassed. Diabodies are bivalent, bispecific antibodies in which VH and VL domains are expressed on a single polypeptide chain, but using a linker that is too short to allow for pairing between the two domains on the same chain, thereby forcing the domains to pair with complementary domains of another chain and creating two antigen binding sites (see e.g., Holliger, P., et al. (1993) Proc. Natl. Aca. Sci. USA 90:6444-6448; Poljak, R.J., et al. (1994) Structure 2:1121-1123.

Still further, an antibody or antigen-binding portion thereof may be part of a larger immunoadhesion molecule, formed by covalent or noncovalent association of the antibody or antibody portion with one or more other proteins or peptides. Examples of such immunoadhesion molecules include use of the streptavidin core region to make a tetrameric scFv molecule (Kipriyanov, S.M., et al. (1995) Human Antibodies and Hybridomas 6:93-101) and use of a cysteine residue, a marker peptide and a C-terminal poly-histidine tag to make bivalent and biotinylated scFv molecules (Kipriyanov, S.M., et al. (1994) Molecular Immunology 31:1047-1058). Antibody portions, such as Fab and F(ab')₂ fragments, can be prepared from whole antibodies using conventional techniques, such as papain or pepsin digestion, respectively, of whole Abs. Moreover, Abs, Ab portions and immunoadhesion molecules can be obtained using standard recombinant DNA techniques, as described herein.

The term "human antibody", as used herein, is intended to include antibodies having variable and constant regions derived from human germline immunoglobulin sequences. The human antibodies of the invention may include amino acid residues not encoded by human germline immunoglobulin sequences (e.g. mutations introduced by random or site-specific mutagenesis *in vitro* or by somatic mutation *in vivo*)*,* for example, in the CDRs and in particular CDR2. However, the term "human antibody", as used herein, is not intended to include antibodies in which CDR sequences derived from the germline of another mammalian species, such as a mouse, have been grafted onto human framework sequences.

The term "recombinant antibody", as used herein, is intended to include all human antibodies that are prepared, expressed, created or isolated by recombinant means, such as antibodies expressed using a recombinant expression vector transfected into a host cell (described further in Section II, below), antibodies isolated from a recombinant, combinatorial human antibody library (Hoogenboom H.R, (1997) TIB Tech. 15:62-70; Azzazy H., and Highsmith W.E., (2002) Clin. Biochem. 35:425-445; Gavilondo J.V., and Larrick J.W. (2002) BioTechniques 29:128-145; Hoogenboom H., and Chames P. (2000) Immunology Today 21:371-378 ), antibodies isolated from an animal (e.g., a mouse) that is transgenic for human immunoglobulin genes (see e.g., Taylor, L. D., et al. (1992) Nucl. Acids Res. 20:6287-6295; Kellermann S-A., and Green L.L. (2002) Current Opinion in Biotechnology 13:593-597; Little M. et al (2000) Immunology Today 21:364-370) or antibodies prepared, expressed, created or isolated by any other means that involves splicing of immunoglobulin gene sequences to other DNA sequences. Such recombinant antibodies have variable and/or constant regions derived from human germline immunoglobulin sequences. In certain embodiments, however, such recombinant antibodies are subjected to *in vitro* mutagenesis (or, when an animal transgenic for human Ig sequences is used, *in vivo* somatic mutagenesis) and thus the amino acid sequences of the VH and VL regions of the recombinant antibodies are sequences that, while derived from and related to germline VH and VL sequences, may not naturally exist within the antibody germline repertoire *in vivo.*

An "isolated antibody", as used herein, is intended to refer to an antibody that is substantially free of other antibodies having different antigenic specificities (e.g., an isolated antibody that specifically binds EpoR is substantially free of antibodies that specifically bind antigens other than EpoR). An isolated antibody that specifically binds EpoR may, however, have cross-reactivity to other antigens, such as EpoR molecules from other species. Moreover, an isolated antibody may be substantially free of other cellular material and/or chemicals.

An "activating or agonistic antibody" or "antibody that activates" or antibody having "activating or agonistic capacity" is intended to refer to an antibody whose binding to EpoR results in stimulation or activation of EpoR biological activity. This biological activity can be assessed by measuring one or more indicators of EpoR biological activity, including but not limited to, antibody induced proliferation of an Epo responsive cell line, antibody induced changes in reticulocyte count and/or percent hematocrit and/or antibody binding to Epo receptors. These indicators of EpoR biological activity can be assessed by one or more of several standard *in vitro or in vivo* assays well known to those of ordinary skill in the art.

The term "chimeric antibody" refers to antibodies which comprise heavy and light chain variable region sequences from one species and constant region sequences from another species, such as antibodies having murine heavy and light chain variable regions linked to human constant regions.

The term "CDR-grafted antibody" refers to antibodies which comprise heavy and light chain variable region sequences from one species but in which the sequences of one or more of the CDR regions of V_{H} and/or VL are replaced with CDR sequences of another species, such as antibodies having murine heavy and light chain variable regions in which one or more of the murine CDRs (*e.g*., CDR3) has been replaced with human CDR sequences.

The term "humanized antibody" refers to antibodies which comprise heavy and light chain variable region sequences from a non-human species (*e.g*., a mouse) but in which at least a portion of the VH and/or VL sequence has been altered to be more "human-like", i.e., more similar to human germline variable sequences. One type of humanized antibody is a CDR-grafted antibody, in which human CDR sequences are introduced into non-human VH and VL sequences to replace the corresponding nonhuman CDR sequences. Means for making chimeric, CDR-grafted and humanized antibodies are known to those of ordinary skill in the art (see, e.g., U.S. Patent Nos. 4,816,567 and 5,225,539). One method for making human antibodies employs the use of transgenic animals, such as a transgenic mouse. These transgenic animals contain a substantial portion of the human antibody producing genome inserted into their own genome and the animal's own endogenous antibody production is rendered deficient in the production of antibodies. Methods for making such transgenic animals are known in the art. Such transgenic animals can be made using XenoMouse® technology or by using a "minilocus" approach. Methods for making Xenomice^{™} are described in U.S. Patent Nos. 6,162,963, 6,150,584, 6,114,598 and 6,075,181. Methods for making transgenic animals using the "minilocus" approach are described in U.S. Patents 5,545,807, 5,545,806 and 5,625,825. Also see International Publication No. WO93/12227.

The term "surface plasmon resonance", as used herein, refers to an optical phenomenon that allows for the analysis of real-time biospecific interactions by detection of alterations in protein concentrations within a biosensor matrix, for example, using the BIAcore system (Pharmacia Biosensor AB, Uppsala, Sweden and Piscataway, New Jersey). For further descriptions, see Example 8 and Jonsson, U., et al. (1993) Ann. Biol. Clin. 51:19-26; Jonsson, U., et al. (1991) Biotechniques 11:620-627; Johnsson, B. et al. (1995) J. Mol. Recognit. 8:125-131; and Johnnson, B., et al. (1991) Anal. Biochem. 198:268-277.

The term "K*_{off}*", as used herein, is intended to refer to the off rate constant for dissocation of an antibody from an antibody/antigen complex.

The term "K*ₒₙ*", as used herein, is intended to refer to the association constant of an antibody to an antigen.

The term "K*_{d}*", as used herein, is intended to refer to the dissociation constant of a particular antibody-antigen interaction. K*_{d}* can be obtained by the following equation: K*_{d}*(M) = K*_{off}*(1/s)/K*ₒₙ*(1/M·s).

The term "polypeptide", as used herein, refers to any polymeric chain of amino acids. The terms "peptide" and "protein" are used interchangeably with the term polypeptide and also refer to a polymeric chain of amino acids. The term "polypeptide" encompasses native or artificial proteins, protein fragments and polypeptide analogs of a protein sequence. A polypeptide may be monomeric or polymeric.

The term "isolated protein" or "isolated polypeptide" is a protein or polypeptide that by virtue of its origin or source of derivation is not associated with naturally associated components that accompany it in its native state; is substantially free of other proteins from the same species; is expressed by a cell from a different species; or does not occur in nature. Thus, a polypeptide that is chemically synthesized or synthesized in a cellular system different from the cell from which it naturally originates will be "isolated" from its naturally associated components. A protein may also be rendered substantially free of naturally associated components by isolation, using protein purification techniques well known in the art.

The term "recovering" as used herein, refers to the process of rendering a chemical species such as a polypeptide substantially free of naturally associated components by isolation, e.g., using protein purification techniques well known in the art.

The term "endogenous activity of EpoR" as used herein, refers to any and all inherent biological properties of the erythropoietin receptor that occur as a consequence of binding of a natural ligand. Biological properties of EpoR include but are not limited to survival, differentiation and proliferation of hematopoeitic cells, an increase in red blood cell production and increase in hematocrit *in vivo.*

The terms "specific binding" or "specifically binding", as used herein, in reference to the interaction of an antibody, a protein, or a peptide with a second chemical species, mean that the interaction is dependent upon the presence of a particular structure (e.g., an antigenic determinant or epitope) on the chemical species; for example, an antibody recognizes and binds to a specific protein structure rather than to proteins generally. If an antibody is specific for epitope "A", the presence of a molecule containing epitope A (or free, unlabeled A), in a reaction containing labeled "A" and the antibody, will reduce the amount of labeled A bound to the antibody.

The term "epitope" includes any polypeptide determinant capable of specific binding to an immunoglobulin or T-cell receptor. In certain embodiments, epitope determinants include chemically active surface groupings of molecules such as amino acids, sugar side chains, phosphoryl, or sulfonyl, and, in certain embodiments, may have specific three dimensional structural characteristics, and/or specific charge characteristics. An epitope is a region of an antigen that is bound by an antibody. In certain embodiments, an antibody is said to specifically bind an antigen when it preferentially recognizes its target antigen in a complex mixture of proteins and/or macromolecules.

The term "polynucleotide" as referred to herein means a polymeric form of two or more nucleotides, either ribonucleotides or deoxyribonucleotides or a modified form of either type of nucleotide. The term includes single and double stranded forms of DNA but preferably is double-stranded DNA.

The term "isolated polynucleotide" as used herein shall mean a polynucleotide (e.g., of genomic, cDNA, or synthetic origin, or some combination thereof) that, by virtue of its origin, the "isolated polynucleotide": is not associated with all or a portion of a polynucleotide with which the "isolated polynucleotide" is found in nature; is operably linked to a polynucleotide that it is not linked to in nature; or does not occur in nature as part of a larger sequence.

The term "vector", as used herein, is intended to refer to a nucleic acid molecule capable of transporting another nucleic acid to which it has been linked. One type of vector is a "plasmid", which refers to a circular double stranded DNA loop into which additional DNA segments may be ligated. Another type of vector is a viral vector, wherein additional DNA segments may be ligated into the viral genome. Certain vectors are capable of autonomous replication in a host cell into which they are introduced (e.g., bacterial vectors having a bacterial origin of replication and episomal mammalian vectors). Other vectors (e.g., non-episomal mammalian vectors) can be integrated into the genome of a host cell upon introduction into the host cell, and thereby are replicated along with the host genome. Moreover, certain vectors are capable of directing the expression of genes to which they are operatively linked. Such vectors are referred to herein as "recombinant expression vectors" (or simply, "expressions vectors"). In general, expression vectors of utility in recombinant DNA techniques are often in the form of plasmids. In the present specification, "plasmid " and "vector" may be used interchangeably as the plasmid is the most commonly used form of vector. However, the invention is intended to include such other forms of expression vectors, such as viral vectors (e.g., replication defective retroviruses, adenoviruses and adeno-associated viruses), which serve equivalent functions.

The term "recombinant host cell" (or simply "host cell"), as used herein, is intended to refer to a cell into which a vector (for example, plasmid, recombinant expression vector) has been introduced. It should be understood that such terms are intended to refer not only to the particular subject cell but also to the progeny of succeeding generations due to either mutation or environmental influences, such progeny may not, in fact, be identical to the parent cell, but are still included within the scope of the term "'host cell" as used herein.

The term "ligand" refers to any chemical moiety capable of binding a polypeptide. Preferably a ligand is an antigen. Antigens may possess one or more epitopes. Ligands to a first polypeptide sequence and second polypeptide sequence may be the same or different.

A "linking sequence" is a polypeptide sequence that connects two or more polypeptide sequences. The term "connects" refers to the joining of polypeptide sequences. Polypeptide sequences are joined preferably by peptide bonding.

### L Antibodies that Bind Human EpoR

The invention provides an isolated antibody, or an antigen-binding portion thereof, that dissociates from human erythropoietin receptor (EpoR) with a K*_{off}* rate constant of greater than about 1.3×10⁻³s⁻¹ and that activates an endogenous activity of said human EpoR in a mammal, said antibody or antigen-binding portion thereof comprising a heavy chain variable region HCVR) and a light chain variable region, wherein the heavy chain variable region comprises an amino acid sequence of Formula I: wherein X₁ is independently selected from the group consisting of tyrosine (Y), glycine (G) and alanine (A); X₂ is independently selected from the group consisting of tyrosine (Y), glycine (G), alanine (A), glutamine (E) and aspartic acid (D); and X₃ is independently selected from the group consisting of serine (S), glycine (G), glutamine (E) and threonine (T) with the proviso that X1-X2-X3 is other than Y-Y-S, and the light chain variable region comprises an amino acid sequence of SEQ ID NO:17. The most preferred recombinant activating antibody of the invention is referred to herein as Ab12.6. The binding properties of Ab12.6 and Ab12.6-related antibodies, all of which are "activating antibodies of EpoR, are summarized in Example 8 below.

The anti-EpoR antibody, and related antibodies, also exhibit a strong capacity to activate EpoR biological activity, as assessed by several *in vitro* and *in vivo* assays (see Examples 9-13). For example, these antibodies activate EpoR in UT-7/Epo cells with EC₅₀ values in the range of about 0.34 nM to 1.345 nM. Ab 12.6 activates EpoR in UT-7/Epo cells with an EC₅₀ of 0.58 nM. Moreover, the activating capacity of the antibodies of the invention are maintained when the antibody is expressed as an Fab, F(ab')₂ or scFv fragment. Furthermore, such antibodies induce an increase in % hematocrit in mammals expressing human EpoR.

Regarding the binding specificity of Ab12.6 and variants thereof, this antibody binds to human EpoR in various forms, including soluble EpoR and transmembrane EpoR. Neither Ab12.6 nor its variants specifically binds to other cytokine receptors.

It is understood in the art that some variability (e.g. up to ±20%) may occur in the calculation of EC₅₀, K*_{off}*, and K*ₒₙ* values based on instrument variation and experimental design. Typically, such measurements are performed using duplicate or triplicate samples to minimize variability. In addition, such an antibody or antigen-binding portion thereof, binds in a manner sufficient to activate human EpoR as demonstrated by a standard *in vitro* proliferation assay.

More preferably, the isolated antibody, or antigen-binding portion thereof, dissociates from human EpoR with an off rate (K*_{off}*) of about 1.4 x 10⁻³ s⁻¹ or greater, more preferably, with a K*_{off}* of about 1.5 x 10⁻³ s⁻¹ or greater, more preferably with a K*_{off}* of about 1.6 x10⁻³ s⁻¹ or greater, more preferably with a K*_{off}* of about 1.7x10⁻³ s⁻¹ or greater, more preferably with a K*_{off}* of about 1.8 x10⁻³ s⁻¹ or greater, and even more preferably, with a K*_{off}* of about 1.9 x10⁻³ s⁻¹ or greater. In a particularly preferred embodiment the isolated human antibody or antigen-binding portion thereof dissociates from human EpoR with a K*_{off}* of about 4.8 x 10⁻³ s⁻¹ or greater Even more preferably, the isolated human antibody, or antigen-binding portion thereof, dissociates from human EpoR an off rate of at least 1.9 x10⁻³ s⁻¹ or at least 4.8x10⁻³ s⁻¹.

In another embodiment, such an antibody or antibody binding portion thereof associates with human EpoR with a K*_{d}* rate constant equal to or greater than about 7 nM and more preferably, with a K*_{d}* rate constant of between about 7-32 nM, inclusive. More preferably, an antibody or antibody binding portion thereof associates with human EpoR with a K*_{d}* rate constract at least equal to 7 nM and up to 32 nM, inclusive. K*_{d}* may be calculated from K*_{off}* and K*ₒₙ* rate constants, which constants may be determined by plasmon surface resonance or other methodologies well know to those of ordinary skill in the art. In a more preferred embodiment an antibody or antigen-binding portion thereof dissociates from EpoR with a K*_{off}* of about 1.9 x10⁻³ s⁻¹ and a K*_{d}* of about 20 nM. In a preferred embodiment, an antibody or antigen-binding portion thereof dissociates from human EpoR with a K*_{off}* of about 4.8x10⁻³ s⁻¹ and a K*_{d}* of about 32 nM. In most preferred embodiments, an antibody or antigen-binding portion thereof dissociates from human EpoR with a K*_{off}* of at least 1.9x10⁻³ s⁻¹ and a K*_{d}* of at least 20 nm. In a preferred embodiment, an antibody or antigen-binding portion thereof dissociates from human EpoR with a K*_{off}* of at least 4.8 x10⁻³ s⁻¹ and a K*_{d}* of at least 32 nM,

More preferably, the isolated antibody, or antigen-binding portion thereof, activates human EpoR in a standard *in vitro* proliferation assay using a human erythroleukemic cell line, such as for example F36E or UT-7/Epo. In a preferred embodiment, the antibody is an isolated human recombinant, antibody, or an antigen-binding portion thereof.

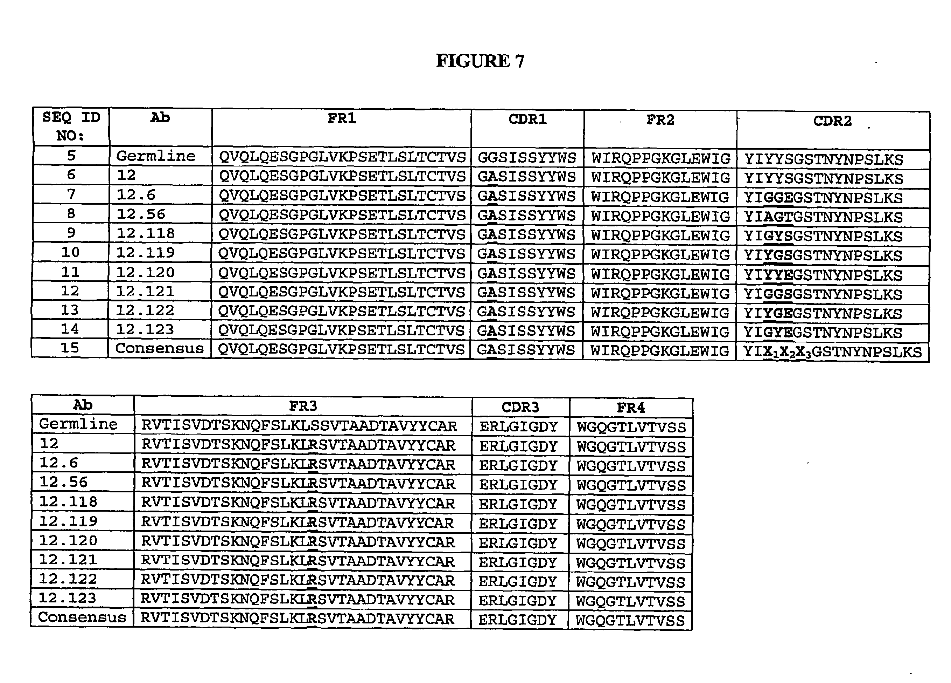

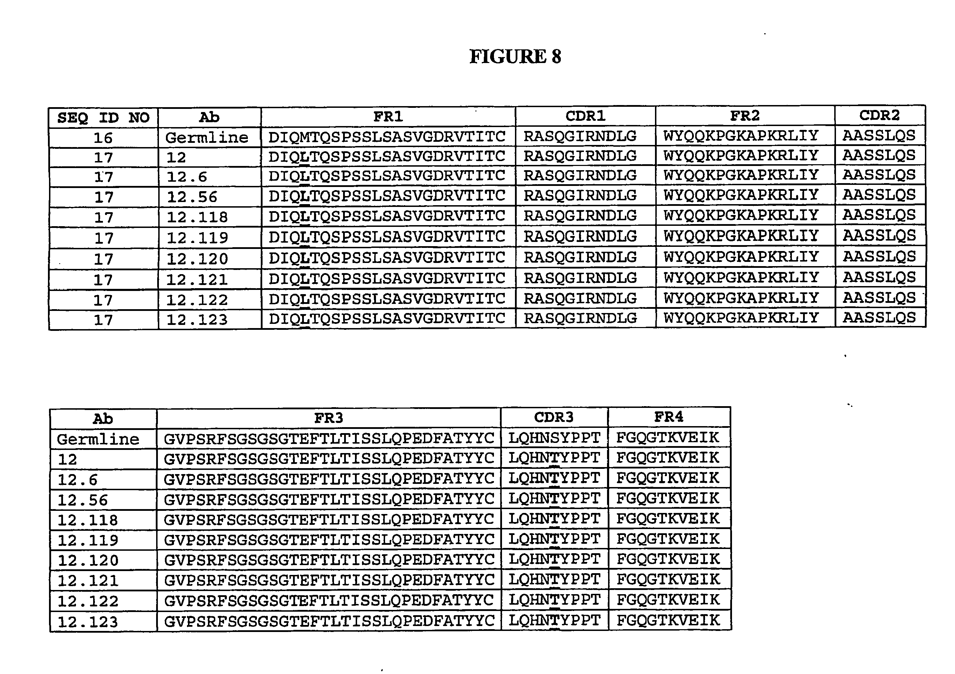

Surface plasmon resonance analysis for determining K*_{d}* and K*_{off}* is well known to those of ordinary skill in the art and can be performed as described herein ( see Example8). A standard *in vitro* assay for determining cell proliferation is described in Example 9. Examples of recombinant human antibodies that meet, or are predicted to meet, the aforementioned kinetic and activation criteria include antibodies having the following [VH/VL] pairs, the sequences of which are shown in FIGS. 7 and 8: SEQ ID NO:15/SEQ ID NO:17, SEQ ID NO:7/SEQ ID NO:17, SEQ ID NO:8/SEQ ID NO:17, SEQ ID NO:9/SEQ ID NO:17, SEQ ID NO:10/SEQ ID NO:17, SEQ ID NO:11/SEQ ID NO:17, SEQ ID NO:12/SEQ ID NO:17, SEQ ID NO:13/SEQ ID NO:17, and SEQ ID NO:14/SEQ ID NO:17.

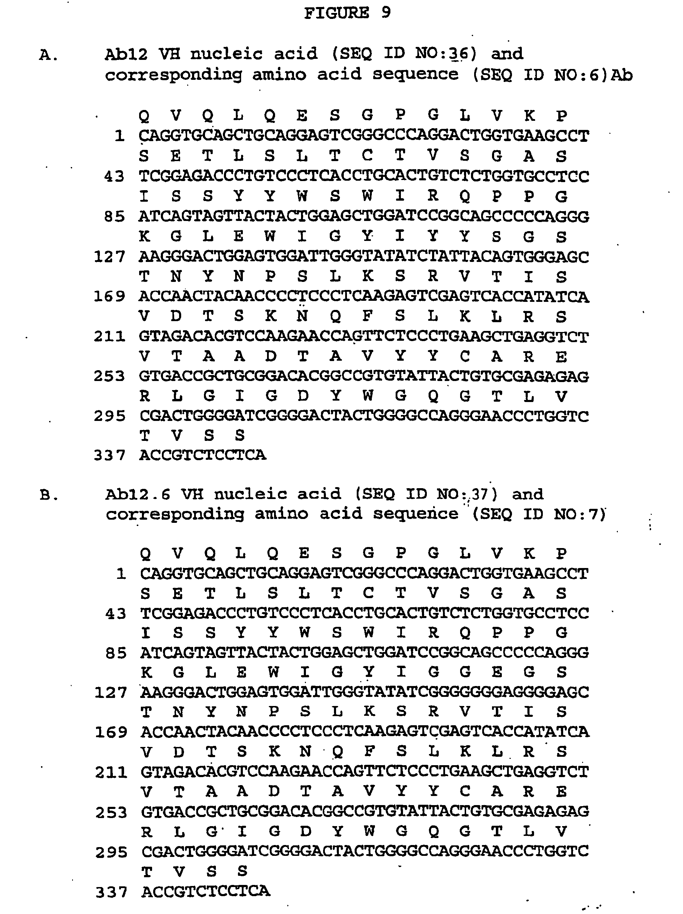

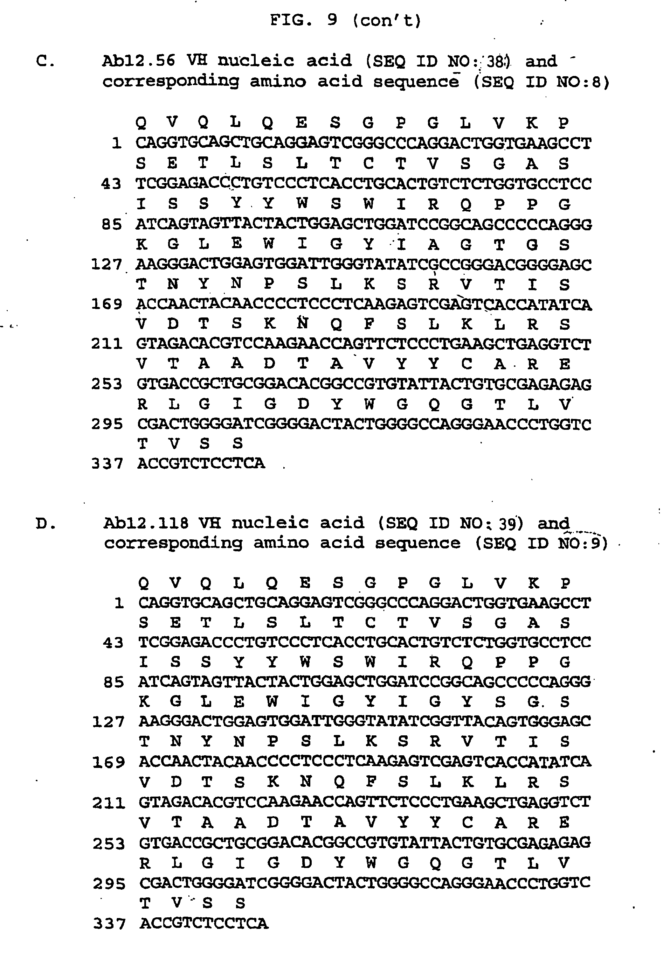

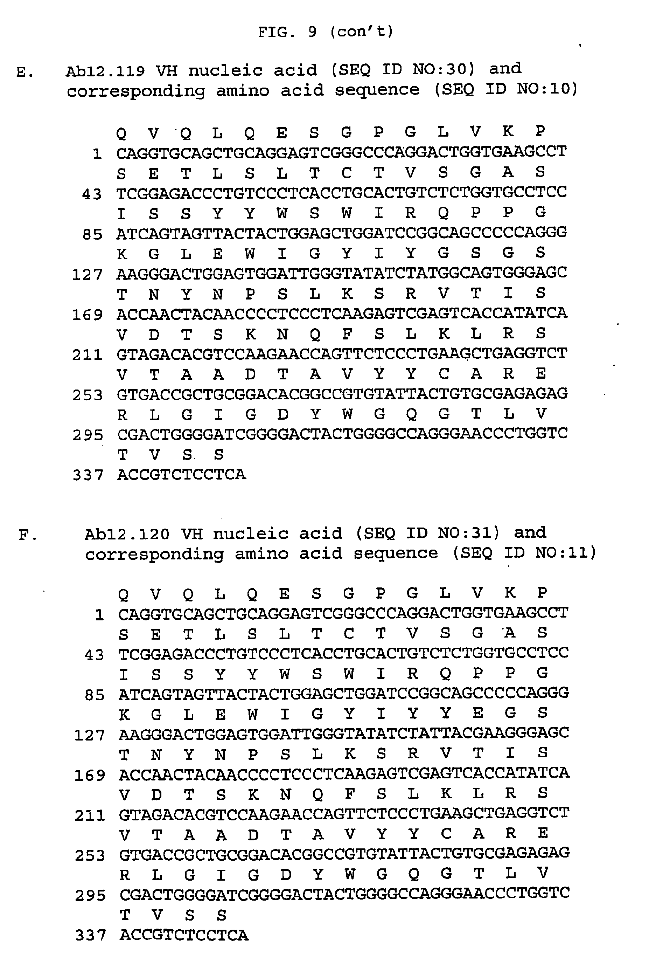

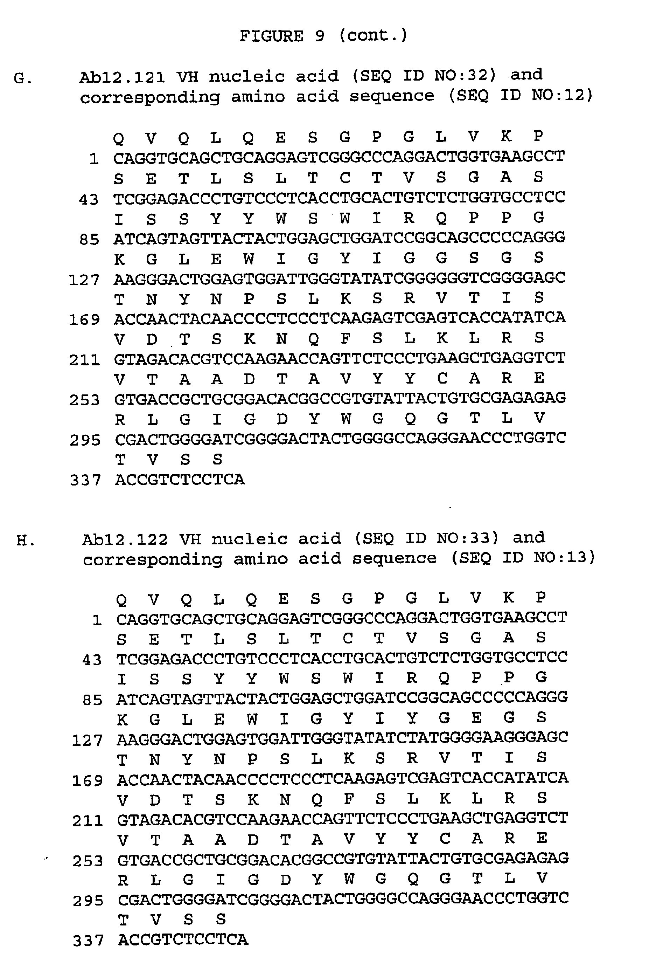

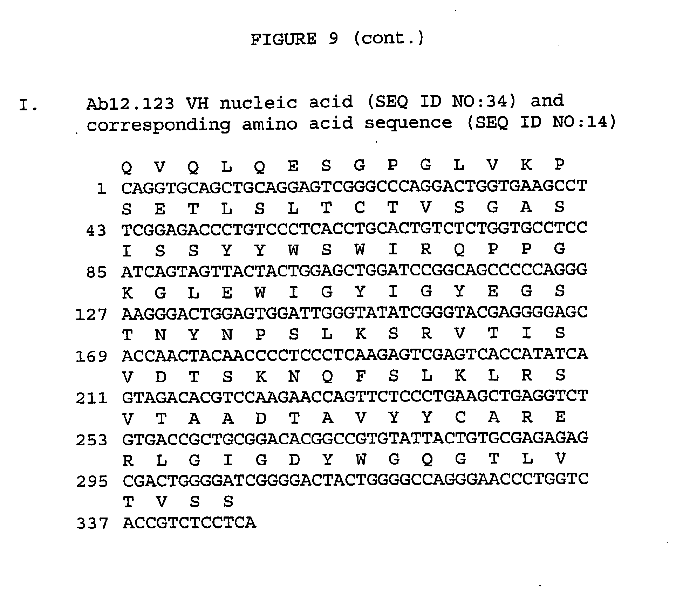

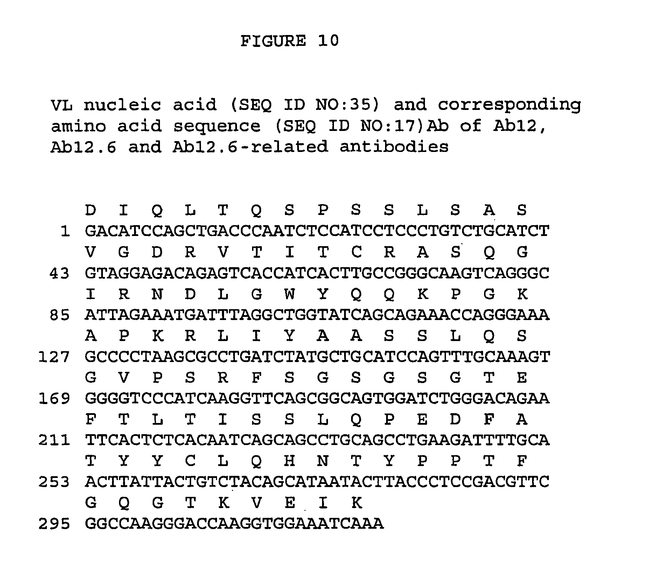

In a preferred embodiment, Ab12.6 and Ab12.6 related antibodies comprise the heavy chain CDR2 sequences shown in Figure 7. In a more preferred embodiment, Ab12.6 and Ab12.6 related antibodies comprise the VE: sequences shown in Figure 9. In an even more preferred embodiment, Ab12.6 and Ab12.6 related antibodies further comprise the VL sequences shown in Figure 10.

### II. Expression of Antibodies

An antibody, or antibody portion, of the invention can be prepared by recombinant expression of immunoglobulin light and heavy chain genes in a host cell. To express an antibody recombinantly, a host cell is transfected with one or more recombinant expression vectors carrying DNA fragments encoding the immunoglobulin light and heavy chains of the antibody such that the light and heavy chains are expressed in the host cell and, preferably, secreted into the medium in which the host cells are cultures, from which medium the antibodies can be recovered. Standard recombinant DNA methodologies are used to obtain antibody heavy and light chain genes, incorporate these genes into recombinant expressions vectors and introduce the vectors into host cells, such as those described in Sambrook, Fritsch and Maniatis (eds), Molecular Cloning: A Laboratory Manual, Second Edition, Cold Spring Harbor, New Your, (1989), Ansubel, F. M. et al. (eds.) Current Protocols in Molecular Biology, Greene Publishing associates (1989) and in U.S. Patent No. 4,816,397 by Boss et al*.*

To express an anti-EpoR antibody of the invention, DNA fragments encoding the light and heavy chain variable regions are first obtained. These DNAs can be obtained by amplification and modification of germline light and heavy chain variable sequences using the polymerase chain reaction (PCR) and as described herein. To express Ab12.6 or an Ab12.6-related antibody, DNA fragments encoding the light and heavy chain variable regions are first obtained. These DNAs can be obtained by amplification and modification of germline light and heavy chain variable sequences using the polymerase chain reaction (PCR). Germline DNA sequences for human heavy and light chain variable region genes are known in the art (see e.g., the "Vbase" human germline sequence database; see also Kabat, B. A, et al. (1991) Sequences of Proteins of Immunological Interest, Fifth Edition, U.S. Department of Health and Human Services, NIH Publication No. 91-3242; Tomlinson, L M., et al. (1992) The Repertoire of Human Germline VH Sequences Reveals about Fifty Groups of V body portion of the invention can be functionally linked (by Segments with Different Hypervariable Loops" J. Mol. Biol. 227:776-798; and Cox, J. P. L. et al. (1994) "A Directory of Human Germ-line V78 Segments Reveals a Strong Bias in their Usage" Eur. J. Immunol. 24:827-836).

To obtain a DNA fragment encoding the heavy chain variable region of Ab12.6, or an Ab12.6-related antibody, the VH4-59 human germline sequence is amplified by standard PCR. In addition, the A30 germline sequence of the Vκ₁ family is amplified by standard PCR. PCR primers suitable for use in amplifying the VH4-59 germline sequence and A30 germline sequence of the Vκ₁ familycan be designed based on the nucleotide sequences disclosed in the references cited supra, using standard methods.

Alternatively, DNA may be obtained from the cell line expressing Ab12 and modified by means well known in the art (such as site-directed mutagenesis) to generate Ab12.6 and Ab12.6-related antibodies. A cell line expressing Ab12 antibody was deposited with the American Type Culture Collection (ATCC), 10801 University Boulevard, Manassas, Virginia 20110, under the terms of the Budapest Treaty, on September 30, 2003 and was accorded accession number PTA-5554. This deposit is provided for the convenience of those skilled in the art and is neither an admission that such deposit is required to practice the invention nor that equivalent embodiments are not within the skill of the art in view of the present disclosure. The public availability of this deposit is not a grant of a license to make, use or sell the deposited material under this or any other patents. The nucleic acid sequence of the deposited material is controlling if in conflict with any sequence described herein.

Once the germline or Ab12 VH and VL fragments are obtained, these sequences can be mutated to encode the Ab12.6 or Ab12.6-related amino acid sequences disclosed herein. The amino acid sequences encoded by the germline or Ab12 VH and VL DNA sequences are first compared to the Ab12.6 or Ab12.6-related VH and VL amino acid sequences to identity amino acid residues in the Ab12.6 or Ab 12.6-related sequence that differ. The appropriate nucleotides of the germline or Ab12 DNA sequences are mutated such that the mutated sequence encodes the Ab12.6 or Ab12.6-related amino acid sequence, using the genetic code to determine which nucleotide changes should be made. Mutagenesis of the germline or Ab 12 sequences is carried out by standard methods, such as PCR-mediated mutageneisis (in which the mutated nucleotides are incorporated into the PCR primers such that the PCR product contains the mutations) or site-directed mutagenesis.

Once DNA fragments encoding Ab12.6 or Ab12.6-related VH and VL segments are obtained (by amplification and mutagenesis of VH and VL genes, as described above), these DNA fragments can be further manipulated by standard recombinant DNA techniques, for example to convert the variable region genes to full-length antibody chain genes, to Fab fragment genes or to a scFv gene. In these manipulations, a VL- or VH-encoding DNA fragment is operatively linked to another DNA fragment encoding another protein, such as antibody constant region or a flexible linker. The term "operatively linked", as used in this context, is intended to mean that the two DNA fragments are joined such that the amino acid sequences encoded by the two DNA fragments remain in-frame.

In an alternative method, an scFv gene may be constructed with wild type CDR regions (eg. of Ab12) and then mutated in the manner described in Examples 3 below.

The isolated DNA encoding the VH region can be converted to a full-length heavy chain gene by operatively linking the VH-encoding DNA to another DNA molecule encoding heavy chain constant regions (CH1, CH2 and CH3). The sequences of human heavy chain constant region genes are known in the art (see e.g., Kabat, E. A., el al. (1991) Sequences of Proteins of Immunological Interest, Fifth Edition, U.S. Department of Health and Human Services, NIH Publication No. 91-3242). The present invention further encompasses all known human heavy chain constant regions, including but not limited to all known allotypes of the human heavy chain constant region. DNA fragments encompassing these regions can be obtained by standard PCR amplification. The heavy chain constant region can be an IgG1, IgG2, IgG3, IgG4, IgA, IgE, IgM or IgD constant region, but most preferably is an IgG2 constant region. For a Fab fragment heavy chain, the VH-encoding DNA can be operatively linked to another DNA molecule encoding only the heavy chain CH1 constant region.

The isolated DNA encoding the VL region can be converted to a full-length light chain gene (as well as a Fab light chain gene) by operatively linking the VL-encoding DNA to another DNA molecule encoding the light chain constant region, CL. The sequences of human light chain constant region genes are known in the art (see e.g., Kabat, E.A., et al. (1991) Sequences of Proteins of Immunological Interest, Fifth Edition, U.S. Department of Health and Human Services, NIH Publication No. 91-3242). The present invention encompasses all known human light chain constant regions, including but not limited to all known allotypes of the human light chain constant region. DNA fragments encompassing these regions can be obtained by standard PCR amplification. The light chain constant region can be a kappa or lambda constant region, but most preferably is a kappa constant region.

It is to be understood that the specific designations of FR and CDR regions within-a particular heavy or-light chain variable region may vary depending on the convention or numbering system used to identify such regions (e.g. Chothia, Kabat, Oxford Molecular's AbM modeling software, all of which are known to those of ordinary skill in the art). Such designations, however, are not critical to the invention.

To create a scFv gene, the VH- and VL-encoding DNA fragments are operatively linked to another fragment encoding a flexible linker, e.g., encoding the amino acid sequence GENKVEYAPALMALS (SEQ ID NO:2) such that the VH and VL sequences can be expressed as a contiguous single-chain protein, with the VL and VH regions joined by, for example, a second flexible linker GPAKELTPLKEAKVS (SEQ ID NO:3). For other linkers sequences also see e.g., Bird et al. (1988) Science 242:423-426, Huston et al. (1988) Proc. Natl. Acad. Sci. USA 85:5879-5883 and McCafferty et al., Nature (1990) 348:552-554.

To express the antibodies, or antibody portions of the invention, DNA's encoding partial or full-length light and heavy chains, obtained as described above, are inserted into expression vectors such that the genes are operatively linked to transcriptional and translational control sequences. In this context, the term "operatively linked" is intended to mean that an antibody gene is ligated into a vector such that transcriptional and translational control sequences within the vector serve their intended function of regulating the transcription and translation of the antibody gene. The expression vector and expression control sequences are chosen to be compatible with the expression host cell used. The antibody light chain gene and the antibody heavy chain gene can be inserted into separate vectors or, more typically, both genes are inserted into the same expression vector. The antibody genes are inserted into the expression vector by standard methods (e.g., ligation of complementary restriction sites on the antibody gene fragment and vector, or blunt end ligation if no restriction sites are present). Prior to the insertion of the Ab12.6 or Ab12.6-related light or heavy chain sequences, the expression vector may already carry antibody constant region sequences. For example, one approach to converting the Ab12.6 or Ab12.6-related VH and VL sequences to full-length antibody genes is to insert them into expression vectors already encoding heavy chain constant and light chain constant regions, respectively, such that the VH segment is operatively linked to the CH "segment" within the vector and the VL segment is operatively linked to the CL segment within the vector. Additionally or alternatively, the recombinant expression vector can encode a signal peptide that facilitates secretion of the antibody chain from a host cell. The antibody chain gene can be cloned into the vector such that the signal peptide is linked in-frame to the amino terminus of the antibody chain gene. The single peptide can be an immunoglobin signal peptide or a heterologous signal peptide (i.e., a signal peptide from a non-immunoglobulin protein).

In addition to the antibody chain genes, the recombinant expression vectors of the invention carry regulatory sequences that control the expression of the antibody chain genes in a host cell. The term "regulatory sequence" is intended to include promoters, enhancers and other expression control elements (e.g., polyadenylation signals) that control the transcription or translation of the antibody chain genes. Such regulatory sequences are described, for example, in Goeddel; Gene Expression Technology. Methods in Enzymology 185, Academic Press, San Diego, Calif. (1990). It will be appreciated by those skilled in the art that the design of the expression vector, including the selection of regulatory sequences may depend on such factors as the choice of the host cell to be transformed, the level of the expression of protein desired, etc. Preferred regulatory sequences for mammalian host cell expression include viral elements that direct high levels of protein expression in mammalian cells, such as promoters and/or enhancers derived from cytomegalovirus (CMV) (such as the CMV promoter/enhancer), Simian Virus 40 (SV40) (such as the SV40 promoter/enhancer), adenovirus, (e.g. the adenovirus major late promoter (AdMLP)) and polyoma. For further description of viral regulatory elements, and sequences thereof, see e.g., U.S. Pat. No. 5,168,062 by Stinski, U.S. Pat. No. 4,510,245 by Bell et al. and U.S. Pat. No. 4,968,615 by Schaffner et al.

In addition to the antibody chain genes and regulatory sequences, the recombinant expression vectors of the invention may carry additional sequences, such as sequences that regulate replication of the vector in host cells (e.g., origins of replication) and selectable marker genes. The selectable marker gene facilitates selection of host cells into which the vector has been introduced (see e.g., U.S.Pat. Nos. 4,399;216, 4,634,665 and 5,179,017, all by Axel et al.). For example, typically the selectable marker gene confers resistance to drugs, such as G418, hygromycin or methotrexate, on a host cell into which the vector has been introduced. Preferred selectable marker genes include the dihydrofolate reductase (DHFR) gene for use in dhfr-host cells with methotrexate selection/amplification and the neomycin (neo) gene for G418 selection.

For expression of the light and heavy chains, the expression vector(s) encoding the heavy and light chains is transfected into a host cell by standard techniques. The various forms of the term "transaction" are intended to encompass a wide variety of techniques commonly used for the introduction of exogenous DNA into a prokaryotic or eukaryotic host cell, e.g., electroporation, calcium-phosphate precipitation, DEAE-dextran transfection. Although it's theoretically possible to express the antibodies of the invention in either prokaryotic or eukaryotic host cells, expression of antibodies in eukaryotic cells, and most preferably mammalian host cells, is the most preferred because such eukaryotic cells, and in particular mammalian cells, are more likely than prokaryotic cells to assemble and secrete a properly folded and immunologically active antibody. Prokaryotic expression of antibody genes has been reported to be ineffective for production of high yields of active antibody (Boss, M. A. and Wood, C. R. (1985) Immunology Today 6:12-13).

Preferred mammalian host cells for expressing the recombinant antibodies of the invention include the Chinese Hamster Ovary (CHO cells) (including dhfr-CHO cells, described in Urlaub and Chasin, (1980) Proc. Natl. Acad. Sci. USA 77:4216-4220, used with a DHFR selectable marker, e.g., as described in R.J. Kaufman and P.A. Sharp (1982) Mol. Biol. 159:601-621), NSO myeloma cells, COS cells, HEK-293 cells, and SP2 cells. When recombinant expression vectors encoding antibody genes are introduced into mammalian host cells, the antibodies are produced by culturing the host cells for a period of time sufficient to allow for expression of the antibody in the host cells or, more preferably, secretion of the antibody into the culture medium in which the host cells are grown. Antibodies can be recovered from the culture medium using standard protein purification methods.

Host cells can also be used to produce portions of intact antibodies, such as Fab fragments or scFv molecules. It will be understood that variations on the above procedure are within the scope of the present invention. For example, it may be desirable to transfect a host cell with DNA encoding either the light chain or the heavy chain (but not both) of an antibody of this invention. Recombinant DNA technology may also be used to remove some or all of the DNA encoding either or both of the light and heavy chains that is not necessary for binding to EpoR. The molecules expressed from such truncated DNA molecules also are encompassed by the antibodies of the invention.

In a preferred system for recombinant expression of an antibody, or antigen binding portion thereof, of the invention, a recombinant expression vector encoding both the antibody heavy chain and the antibody light chain is introduced into dhfr-CHO cells by calcium phosphate-mediated transfection. Within the recombinant expression vector, the antibody heavy and light chain genes are each operatively linked to CMV enhancer/AdMLP promoter regulatory elements to drive high levels of transcription of the genes. The recombinant expression vector also carries a DHFR gene, which allows for selection of CHO cells that have been transfected with the vector using methotrexate selection/amplification. The selected transformant host cells are culture to allow for expression of the antibody heavy and light chains and intact antibody is recovered from the culture medium. Standard molecular biology techniques are used to prepare the recombinant expression vector, transfect the host cells, select for transformants, culture the host cells and recover the antibody from the culture medium.

In view of forgoing, another aspect of the invention pertains to nucleic acid, vector and host cell compositions that can be used for recombinant expression of the antibodies and antibody portions of the invention. The nucleotide sequence encoding the heavy chain variable region of Ab12.6 and variants thereof is shown in FIG. 9. The nucleotide sequence encoding the Ab12.6 light chain variable region is shown in FIG. 10. The CDR1 domain of the HCVR of Ab12.6 encompasses nucleotides 26-35 of SEQ ID NO:7, the CDR2 domain encompasses nucleotides 50-65 of SEQ ID NO:7 and the CDR3 domain encompasses nucleotides 98-105 of SEQ ID NO:7. It will be appreciated by the skilled artisan that nucleotide sequences encoding Ab12.6-related antibodies, or portions thereof (e.g., a CDR domain, such as a CDR2 domain), can be derived from the nucleotide sequences encoding the Ab12.6 LCVR and HCVR using the genetic code and standard molecular biology techniques.

In one embodiment, the invention provides an isolated nucleic acid encoding a heavy chain variable region comprising an amino acid sequence of Formula I: wherein:
X₁ is Independently selected from the group consisting oftyrosine (Y), glycine (0) and alanine (A);
X₂ is independently selected from the group consisting of tyrosine (Y), glycine (G), alanine (A), glutamine (E) and aspartic acid (D); and
X₃ is independently selected from the group consisting of serine (S), glycine (G), glutamime (E) and threonine (T)
with the proviso that X₁-X₂-X₃ is other than Y-Y-S.

In yet another embodiment, the invention provides isolated nucleic acids encoding a polypeptide selected from the group consisting of: SEQ ID NO:7, SEQ ID NO:8, SEQ ID NO:9, SEQ ID NO:10, SEQ ID NO:11, SEQ ID NO:12, SEQ ID NO:13 and SEQ ID NO:14.

In still another embodiment, the invention provides an isolated nucleic acid encoding antibody light chain variable region comprising the amino acid sequence of SEQ ID NO:17. The nucleic acid can encode only the HCVR or can also encode an antibody light chain constant region, operatively linked to the LCVR. In one embodiment, this nucleic acid is in a recombinant expression vector. Those of ordinary skill in the art will appreciate that the nucleic acids encoding the antibodies of the invention are not limited to those specifically described herein but also include, due to the degeneracy of the genetic code, any DNAs which encode the polypeptide sequences described herein. The degeneracy of the genetic code is well established in the art. (See, e.g. Bruce Alberts et al. (eds), Molecular Biology of the Cell, Second Edition, 1989, Garland Publishing Inc., New York and London) Accordingly, the nucleotide sequences of the invention include those comprising any and all degenerate codons at any and all positions in the nucleotide, provided that such codons encode the amino acids sequences as set forth herein.

In still another embodiment, the invention provides an isolated nucleic acid encoding an antibody light chain variable region comprising the amino acid sequence of SEQ ID NO:17 (i.e., the Ab12.6 LCVR although the skilled artisan will appreciate that due to the degeneracy of the genetic code, other nucleotide sequences can encode the amino acid sequence of SEQ ID NO: 17. The nucleic acid can encode the LCVR operatively linked to the HCVR. For example, the nucleic acid can comprise an IgG1, or IgG2 or IgG4 constant region. In a preferred embodiment, the nucleic acid comprises an IgG2 constant region. In yet another embodiment, this nucleic acid is in a recombinant expression vector.

The invention also provides recombinant expression vectors encoding both an antibody heavy chain and an antibody light chain. For example, in one embodiment, the invention provides a recombinant expression vector encoding:
a) an antibody heavy chain having a variable region comprising the amino acid sequence of SEQ ID NO: 7 (i.e., the Ab12.6 HCVR); and
b) an antibody light chain having a variable region comprising the amino acid sequence of SEQ ID NO: 17 (i.e., the Ab12.6 LCVR).

The invention also provides host cells into which one or more of the recombinant expression vectors of the invention have been introduced. Preferably, the host cell is a mammalian host cell, more preferably the host cell is a CHO cell, an NSO cell or an HEK-293 cell or a COS cell. Still further the invention provides a method of synthesizing a recombinant human antibody of the invention by culturing a host cell of the invention in a suitable culture medium until a recombinant human antibody of the invention is synthesized. The method can further comprise isolating the recombinant human antibody from the culture medium.

### III. Selection of Recombinant Antibodies

Recombinant antibodies of the invention in addition to the Ab12.6 or Ab12.6-related antibodies disclosed herein can be isolated by screening of a recombinant combinatorial antibody library, preferably a scFv yeast display library, prepared using chimeric, humanized or human (e.g. Ab12) VL and VH cDNAs. Methodologies for preparing and screening such libraries are known in the art. In addition to commercially available vectors for generating yeast display libraries (e.g.,pYD1 vector, Invitrogen, Carlsbad, California) examples of methods and reagents particularly amenable for use in generating and screening antibody display libraries can be found in, for example, Boder E.T. and Wittrup K.D., Yeast surface display for directed evolution of protein expression, affinity, and stability, Methods Enzymol., 328:430-44 (2000) and Boder E.T. and Wittrup K.D., Yeast surface display for screening combinatorial polypeptide libraries, Nat Biotechnol. 15(6):553-7 (June 1997).

In a preferred embodiment, to isolate human antibodies with low affinity and a fast off-rate for EpoR, a human agonist antibody (such as, for example, Ab12) is first used to generate human heavy and light chain sequences expressed as scFvs on the surface of yeast (preferably *Saccaromyces cerevisiae*)*.* Ab12 scFvs are analyzed to determine those having the highest expression levels. Such constructs then are screened, preferably using soluble recombinant human EpoR. Those scFv constructs having the highest degree of binding of soluble EpoR are selected for subsequent mutagenesis of the heavy and light chain variable regions to generate CDR mutagenic libraries.

To further increase the off-rate constant for EpoR binding, the VH and VL segments of the preferred VH/VL pair(s) can be randomly mutated, preferably within the CDR2 region of VH, in a process analogous to the *in vivo* somatic mutation process responsible for affinity maturation of antibodies during a natural immune response. This *in vitro* affinity maturation can be accomplished by replacing a portion of each CDR with a degenerate single-stranded oligonucleotide encoding three amino acids within the CDR being targeted. The replacement of a portion of each CDR with a new randomized sequence (up to 8000 possibilities) can be accomplished by homologous recombination in yeast (see, e.g. Example 3). These randomly mutated VH segments can be analyzed for binding to EpoR in the context of an scFv; scFvs exhibiting an improved fluorescence and a fast off-rate can then be isolated and the CDR mutation identified by sequencing.

Following screening of a recombinant scFv display library, clones having the desired characteristics are selected for conversion, preferably to immunoglobulin gamma type 2/kappa light chain (IgG2/K) antibodies. Nucleic acid encoding the selected antibody can be recovered from the display package (e.g., from the yeast expression vector) and subcloned into other expression vectors by standard recombinant DNA techniques. If desired, the nucleic acid can be further manipulated to create other antibody forms of the invention (e.g., linked to nucleic acid encoding additional immunoglobulin domains, such as additional constant regions). To express a recombinant human antibody isolated by screening of a combinatorial library, the DNA encoding the antibody is cloned into a recombinant expression vector and introduced into a mammalian host cells, as described in further detail in Section II above.

### IV. Uses of Anti-EpoR Antibodies

The antibodies or antigen-binding portion thereof, of the present invention have a number of uses. In general, the antibodies or antigen-binding portion thereof may be used to treat any condition treatable by erythropoietin or a biologically active variant or analog thereof. For example, antibodies or antigen-binding portions thereof, of the invention are useful for treating disorders characterized by low red blood cell levels and/or decreased hemoglobin levels (e.g. anemia). In addition, such antibodies or antigen-binding portions thereof may be used for treating disorders characterized by decreased or subnormal levels of oxygen in the blood or tissue, such as, for example, hypoxemia or chronic tissue hypoxia and/or diseases characterized by inadequate blood circulation or reduced blood flow. Antibodies or antigen-binding portions thereof also may be useful in promoting wound healing or for protecting against neural cell and/or tissue damage, resulting from brain/spinal cord injury, stroke. Non-limiting examples of conditions that may be treatable by the antibodies of the invention include anemia, such as chemotherapy-induced anemia, cancer associated anemia, anemia, of chronic disease, HIV-associated anemia, bone marrow transplant-associated anemia, and the like, heart failure, ischemic heart disease and renal failure. As such, the antibodies of the invention may be used for treating any of the aforementioned diseases or conditions by administering to a mammal a therapeutically effective amount of said antibody. Preferably, the mammal is a human.

The antibodies or an autigen-binding portions thereof, of the present invention also can be used to identify and diagnose mammals that have a dysfunctional EPO receptor. Mammals that have a dysfunctional EPO receptor are characterized by disorders such as anemia. Preferably, the mammal being identified and diagnosed is a human. Additionally, the antibodies of the present invention can be used in the treatment of anemia in mammals suffering from red blood cell aplasia. Red blood cell aplasia may result from the formation of neutralizing anti-erythropoietin antibodies in patients during treatment with recombinant erythropoietin (Casadevall, N. et aL, n. Eng. J. Med. 346: 469 (2002)). The antibodies of the invention may be used to treat aplasia by administering to a mammal suffering from said aplasia and in need of treatment a therapeutically effective amount of the antibodies of the present invention.

In one embodiment of the invention, the EPO receptor antibodies and antigen-binding portions thereof also can be used to detect EPO receptor (e.g., in a biological sample, such as tissue specimens, intact cells, or extracts tltereof), using a conventional immmoassay, such as an enzyme linked immunosorbent assay (ELISA), a radioimmunoassay (RIA) or tissue immunohistochemistry. The invention provides a method for detecting EPO receptor in a biological sample comprising contacting a biological sample with an antibody or antigen-binding portion of the invention and detecting either the antibody (or antibody portion), to thereby detect EPO receptor in the biological sample. The antibody or antigen-binding portion directly or indirectly labeled with a detectable substance to facilitate detection of the bound or unbound antibody or antibody fragment A variety of immunoassay formats may be practiced (such as competitive assays, direct or indirect sandwich immunoassays) and are well known to those of ordinary skill in the art.

Suitable detectable substances include various enzymes, prosthetic groups, fluorescent materials, luminescent materials and radioactive materials. Examples of suitable enzymes include horseradish peroxidase, alkaline phosphatase, B-galactosidase, or acetylcholinesterase; examples of suitable prosthetic group complexes include steptavidin/biotin and avidin/biotin; examples of suitable fluorescent materials include umbelliferone, fluorescein, fluorescein isothiocyanate, rhodamine, dichlorotriazinylamine, dansyl chloride or phycoerythrin; and an example of a luminescent material includes luminol; and examples of suitable radioactive material include ¹²⁵I, ¹³¹I, ³⁵S. or ³H. Given their ability to bind to human EpoR, the anti-EpoR antibodies, or portions thereof, of the invention can be used to activate or stimulate EpoR activity. The antibodies and antigen-binding portions thereof preferably are capable of activating EpoR activity both *in vitro* and *in vivo*. Accordingly, such antibodies and antibody portions can be used to activate EpoR activity, *e.g*., in a cell culture containing EpoR, in human subjects or in other mammalian subjects having EpoR with which an antibody of the invention cross-reacts.

In another embodiment, the invention provides the antibody or antigen-binding portion thereof of the present disclosure for activating an endogenous activity of a human erythropoietin receptor for treating anemia, hypoxemia, chronic tissue hypoxia, heart failure, ischemic heart disease, renal failure, neural cell damage or tissue damage, or for promoting wound healing in a mammal, by administering to said mammal a therapeutically effective amount of an antibody or antigen-binding portion thereof, of the invention. Preferably, the mammal is a human subject.

An antibody of the invention can be administered to a human subject for therapeutic purposes. Moreover, an antibody of the invention can be administered to a non-human mammal with which the antibody is capable of binding for veterinary purposes or as an animal model of human disease. Regarding the latter, such animal models may be useful for evaluating the therapeutic efficace of antibodies of the invention (*e.g*., testing of dosages and time courses of administration).

In another aspect, the invention provides the antibody or antigen-binding portion thereof of the present disclosure for treating a mammal suffering from aplasia, by administering to the mammal in need of treatment a therapeutically effective amount of an antibody or antigen-binding portion thereof, of the invention. In addition, the invention provides the antibody or antigen-binding portion thereof of the present disclosure for treating a mammal suffering from anemia by administering to the mammal in need of treatment a therapeutically effective amount of an antibody or antigen-binding portion thereof, of the invention.

### V. Pharmaceutical Compositions and Pharmaceutical Administration

The antibodies and antibody-portions of the invention can be incorporated into pharmaceutical compositions suitable for administration to a subject Typically, the pharmaceutical composition comprises a therapeutically or pharmaceutically affective amount of an antibody or antibody portion of the invention along with a pharmaceutically acceptable carrier or excipient. As used herein, "pharmaceutically acceptable carrier" or "pharmaceutically acceptable excipient" includes any and all solvents, dispersion media, coating, antibacterial and antifungal agents, isotonic and absorption delaying agents, that are physiologically compatible. Examples of pharmaceutically acceptable carriers or excipients include one or more of water, saline, phosphate buffered saline, dextrose, glycerol, ethanol as well as combinations thereof. In many cases, it will be preferable to include isotonic agents, for example, sugars, polyalcohols such as mannitol, sorbitol, or sodium chloride in the composition. Pharmaceutically acceptable substances such as wetting or minor amounts of auxiliary substances such as wetting or emulsifying agents, preservatives or buffers, which enhance the shelf life or effectiveness of the antibody or antibody portion also may be included. Optionally, disintegrating agents can be included, such as cross-linked polyvinyl pyrrolidone, agar, alginic acid or a salt thereof, such as sodium alginate. In addition to the excipients, the pharmaceutical composition can include one or more of the following, carrier proteins such as serum albumin, buffers, binding agents, sweeteners and other flavoring agents; coloring agents and polyethylene glycol.

The compositions of this invention may be in a variety of forms. They include, for example, liquid, semi-solid and solid dosage forms, such as liquid solutions (e.g. injectable and infusible solutions), dispersions or suspensions, tablets, pills, powders, liposomes and suppositories. The preferred form depends on the intended mode of administration and therapeutic application. Typical preferred composition are in the form of injectable or infusible solutions, such as compositions similar to those used for passive immunization of humans with other antibodies. The preferred mode of administration is parenteral (e.g, intravenous, subcutaneous, intraperitoneal, intramuscular). In a preferred embodiment, the antibody is administered by intravenous infusion or injection. In another preferred embodiment, the antibody or antibody fragment is administered by intramuscular or subcutaneous injection.

Therapeutic compositions typically must be sterile and stable under the conditions of manufacture and storage. The composition can be formulated as a solution, microemulsion, dispersion, liposome, or other ordered structure suitable to high drug concentration. Sterile injectable solutions can be prepared by incorporating the active compound (i.e. antibody or antibody fragment) in the required amount in an appropriate solvent with one or a combination of ingredients enumerated above, as required, followed by filtered sterilization. Generally, dispersions are prepared by incorporating the active compound into a sterile vehicle that contains a basic dispersion medium and the required other ingredients from those enumerated above. In the case of sterile powders for the preparation of sterile injectable solutions, the preferred methods of preparation are vacuum drying and freeze-drying that yields a powder of the active ingredient plus any additional desired ingredient from a previously sterile-filtered solution thereof. The proper fluidity of a solution can be maintained, for example, by the use of a coating such as lecithin, by the maintenance or the required particle size in the case of dispersion and by the use of surfactants Prolonged absorption of injectable compositions can be brought about by including in the composition an agent that delays absorption, for example, monostearate salts and gelatin.

The antibodies and antibody portions of the invention can be administered by a variety of methods known in the art, although for many therapeutic applications, the preferred route/mode of administration is intravenous induction or infusion. As will be appreciated by the skilled artisan, the route and/or mode of administration will vary depending upon the desired results. In certain embodiments, the active compound may be prepared with a carrier that will protect the compound against rapid release, such as a controlled release formulation, including implants, transdermal patches, and microencapsulated delivery systems. Biodegradable, biocompatible polymers can be used, such as ethylene vinyl acetate, polyanhydrides, polyglycolic acid, collagen, polyorthoesters, and polylactic acid. Many methods for the preparation of such formulations are patented or generally known to those skilled in the art. (See, e.g. Sustained and Controlled Release Drug Delivery Systems, J. R. Robinson, ed, Marcel Dekker, Inc., New York. 1978).

In certain embodiments, an antibody or antibody portion of the invention may be orally administered, for example, with an inert diluent or an assimilable edible carrier. The compound (and other ingredients if desired) may also be enclosed in a hard or soft shell gelatin capsule, compressed into tablet, buccal tablets, troches, capsules, elixiers, suspensions, syrups, wafers. To administer an antibody or antibody fragment of the invention by other than parenteral administration, it may be necessary to coat the compound with, or co-administer the compound with, a material to prevents its inactivation.

Supplementary active compounds also can be incorporated into the compositions. In certain embodiments, the antibody or antibody portion is co-formulated with and/or co-administered with one or more additional therapeutic agents. Such combination therapies may advantageously utilize lower dosages of the administered therapeutic agents, thus avoiding possible toxicities or complications associated with monotherapies or alternatively, act synergistically or additively to enhance the therapeutic effect.

As used herein, the term "therapeutically effective amount" or "pharmaceutically effective amount" means an amount of antibody or antibody portion effective, at dosages and for periods of time necessary, to achieve the desired therapeutic result. The exact dose will be ascertainable by one skilled in the art. As known in the art, adjustments based on age, body weight, sex, diet, time of administration, drug interaction and severity of condition may be necessary and will be ascertainable with routine experimentation by those skilled in the art. A therapeutically effective amount is also one in which the therapeutically beneficial effects outweigh any toxic or detrimental effects of the antibody or antibody fragment. A "prophylactically effective amount" refers to an amount effective, at dosages and for periods of time necessary to achieve the desired prophylactic result. Typically, since a prophylactic dose is used in subjects prior to or at an earlier stage of disease, the prophylactically effective amount will be less than the therapeutically effective amount.

Dosage regimens may be adjusted to provide the optimum desired response (e.g., a therapeutic or prophylactic response). For example, a single bolus may be administered, several divided doses may be administered over time or the dose may be proportionally reduced or increased as indicated by the exigencies of the therapeutic situation. It is especially advantageous to formulate parenteral compositions in dosage unit form for ease of administration and uniformity of dosage. Dosage unit form as used herein refers to physically discrete units suited as unitary dosages for the mammalian subjects to be tested; each unit containing a predetermined quantity of active compound calculated to produce the desired therapeutic effect in association with the required pharmaceutical carrier. The specification for the dosage unit forms of the invention are dictated by and directly dependent on (a) the unique characteristics of the active compound and the particular therapeutic or prophylactic effect to be achieved and (b) the limitations inherent in the art of compounding such an active compound for the treatment of sensitivity in individuals.

An exemplary, non-limiting range for a therapeutically or prophylactically effective amount of an antibody or antibody portion of the invention is 0.1-20 mg/kg, more preferably 0.5-10 mg/kg. It is to be noted that dosage values may vary with the type and severity of the condition to be alleviated. It is to be further understood that for any particular subject, specific dosage regimens should be adjusted over time according to the individual need and the professional judgment of the person administering or supervising the administration of the compositions, and that dosage ranges set forth herein are exemplary only and are not intended to limit the scope or practice of the claimed composition.

### EXAMPLES

### Example 1: Conversion of Ab12 into a single chain antibody fragment

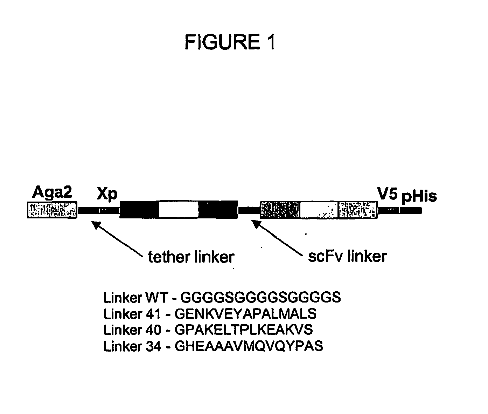

The initial objective of this study was to decrease the off-rate of Ab12 IgG2/K using yeast display technology. To meet this objective, Ab12 IgG2/K was converted into an scFv using linker sequences. Several different linker sequences were scrutinized during the construction of Ab12 scFv (FIG. 1). Each linker combination was assessed by analysis of expression of Ab12 scFv on the surface of yeast (*Saccaromyces. cerevisiae*)*.* The linker combination resulting in the highest surface expression of Ab12 scFv was chosen as the construct to use for subsequent mutagenesis of CDR regions and fluorescence-activated cell sorting (FACS). FIG. 1 represents a schematic depiction of an scFv construct, showing the location of the tether and scFv linkers and the choice of available sequences. Linker sequences were combined in various orders to obtain the highest scFv expression on the surface of yeast.

Various single stranded oligonucleotides encoding Ab12 scFvs were co-transformed with a linearized "gapped" vector derived from pYD1 (Invitrogen, Carlsbad, CA) into yeast by techniques well known to practitioners in the art. Functional cell surface protein expression was compared by incubating the transformed yeast with increasing concentrations of soluble EpoR (EposR) at 37°C (FIG. 2). Bound antigen was detected using a monoclonal antibody to EpoR, MAB307 obtained commercially from R and D Systems (Minneapolis, MN) followed by anti-mouse phycoerythrin (PE, Southern Biotech, Birmingham, AL). The Ab12 scFv construct which showed the highest expression used linker 41 (SEQ ID NO:2) as the tether linker and linker 40 (SEQ ID NO:3) as the scFv linker (hereinafter Ab12 41/40). This construct was used in all subsequent FACS experiments as described below.

### Example 2: Off-rate analysis of Ab12 scFv on yeast

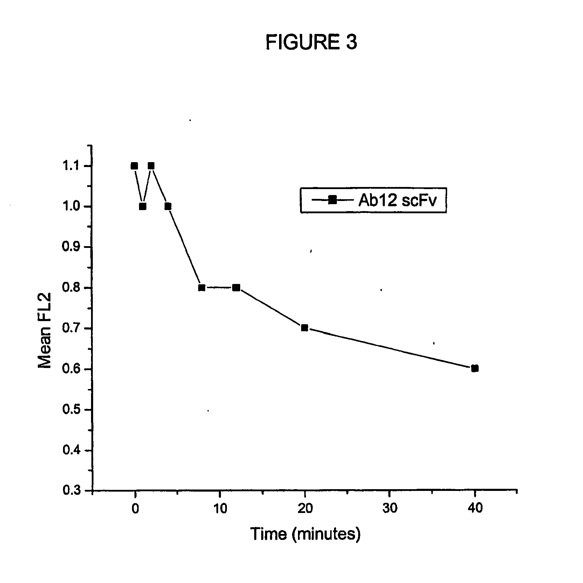

Off-rate measurements of Ab12 41/40 scFv were performed by incubation of 0.5 µM EposR with 0.1 O.D. yeast (approximately 1x10⁶ yeast cells) for 1.5 hours at 37°C; following this cells were chilled on ice and washed at 4°C. A 10,000 fold excess of Ab12 IgG1 (Abbott Laboratories, Abbott Park, IL), warmed to 37°C, was added to the cells and individual samples were withdrawn at various time points, chilled and later read on an Epics XL1 flow cytometer (Beckman Coulter, Fullerton, CA). The experiment was designed so that as EposR dissociated from Ab12 scFv, it would immediately bind to Ab12 IgG1 (present at a saturating concentration) and would no longer be detected on the surface of yeast. The remaining bound EposR was detected by addition of MAB307 followed by addition of anti-mouse PE. Figure 3 represents an off-rate analysis of Ab12 41/40 scFv. As FIG. 3 shows, the bulk of EposR had dissociated by 20 minutes of competition, and this parameter was factored into the off-rate FACS discussed below.

### Example 3: Creation of Ab12 scFv CDR mutagenic libraries

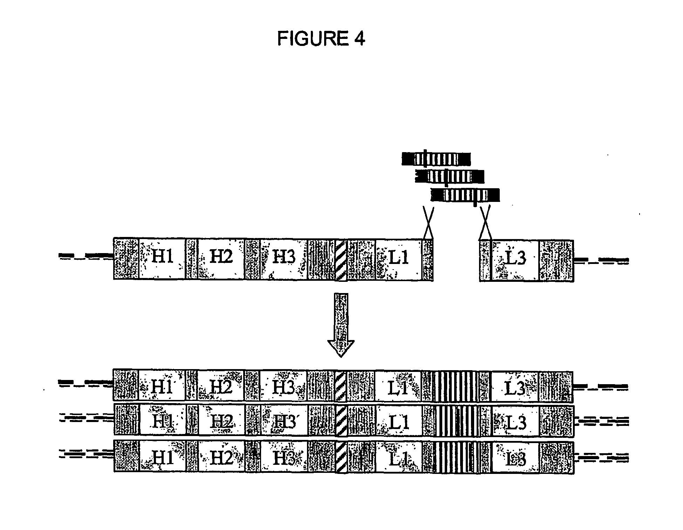

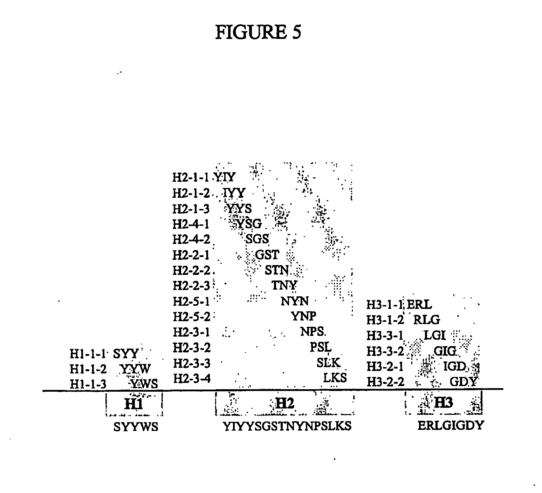

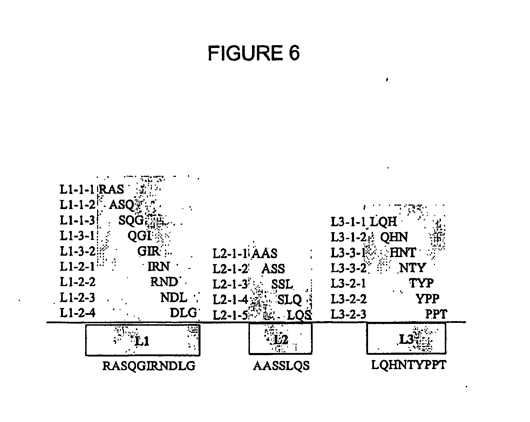

All 6 CDRs of Ab12 41/40 (three in the heavy chain and three in the light chain) were subjected to randomization, and libraries composed of 8000 members each were generated. Linerarized "gapped" pYD1 vector (Invitrogen) was modified to include a TEV protease site and also to contain Ab12 41/40 scFv sequence (i.e. pYD1Tev-Ab12- 41/40). Thereafter, gapped pYD1-Tev-Ab12-41/40 vectors, missing specific regions of each CDR were prepared by PCR, and the gap was replaced by a degenerate single-stranded oligonucleotide encoding three amino acids within the CDR being targeted. The replacement of a portion of each CDR with a new randomized sequence (up to 8000 possibilities) was accomplished by homologous recombination in yeast. A schematic of this library construction method is shown in Figure 4, indicating that gapped vector and single-stranded oligonucleotide are co-transformed into yeast. Gapped vector and oligonucleotide undergo homologous recombination, thereby generating a library of randomized CDRs. A total of 50 libraries were generated using this method. The libraries are shown schematically in Figures 5 and 6.

### Example 4: FACS of Ab12 41/40 scFv Libraries

All 50 Ab12 scFv libraries and wild type Ab12 scFv yeast were subjected to off-rate FACS analysis on a MoFlo high-speed cell sorter. (Dako Cytomation California Inc. Carpinteria, CA) Transformed yeast cells (0.6 O.D.) were incubated with 0.5µM EposR at 37°C until equilibrium was reached (2 hours). Cells were then chilled, washed, and a 10,000 fold molar excess (5 µg/mL) of Ab12 IgG1prewarmed to 37°C was added. After a 20-minute incubation at 37°C, cells were again chilled, washed and labeled depending on whether they were being prepared for "one-color" FACS or "two-color" FACS. For the former, cells were labeled with a mixture of MAB307 and anti-mouse PE. For the latter, cells were labeled first with a mixture of MAB307 and rabbit anti-6-his antibody (Research Diagnostics, Flanders, NJ), followed by a mixture of anti-mouse PE and goat anti-rabbit FITC (Southern Biotech, Birmingham, AL). Individual control samples were also prepared to set MoFlo compensation and to ensure no non-specific background staining existed.

For Round 1 off-rate FACS, each library sample was compared to Ab12 scFv yeast (WT control) for evidence of a population of cells having an increased FL2 fluorescence (and, therefore, a potentially longer off-rate). In each case, the brightest 1% of cells in the FL2 axis were gated, collected, and re-grown in media (Round 1 output). For Round 2 off-rate FACS, the identical cell incubation procedure was performed on each Round 1 library output for some libraries; for others, the Round 2 FACS involved additional reagents to detect surface expression. For each Round 2 off-rate FACS analysis, a gate was drawn around the top 0.1% of cells in the FL2 axis, and this gate was superimposed on all Round 1 library outputs, where applicable. Libraries displaying a population of cells having a higher FL2 than those in the WT gate were selected for FACS, those with no cells inside of the reference gate were not analyzed further. For those selected libraries, the brightest 0.1% of cells in the FL2 axis were gated and collected. An aliquot was plated on selective media for yeast (SD or "single dropout") for yeast colony isolation and the remainder were grown as liquid cultures for future cell analysis.

### Example 5: Analysis of Isolated Clones following Off-rate Sorting

Selected bulk Round 2 outputs were grown in liquid media and subjected to off-rate analysis (data not shown). Outputs displaying improved off-rate curves were chosen for further analysis. Individual clones from these outputs were recovered following plating on selective media and plasmid DNA isolation. PCR was used to amplify the scFv region of each clone and products were sequenced to identify the amino acid substitutions. Table 1 highlights sequencing results from each Round 2 output. All unique clones were named and the frequency of their prevalence noted.

**Table 1**

| | | |
|---|---|---|
| **Ab12 CDRH2 Sequence** Y I Y Y S G S T N Y N P S L K S | | |

| **Library name and sequenced clone #** | **Future IgG2/K name** | **CDR sequence substituted in mutagneic library** |
|---|---|---|
| | | |
| **H2-1-1 WT** | | **Y I Y** |
| H2-1-1 R2 #1,8 | Ab12.26 | Y V G |
| H2-1-1 R2 #2 | Ab12.27 | Y A S |
| H2-1-1 R2 #3 | Ab12.28 | R V G |
| H2-1-1 R2 #4 | Ab12.29 | V R A |
| H2-1-1 R2 #5 | Ab12.30 | K C G |
| H2-1-1 R2 #6 | Ab12.31 | G V G |
| H2-1-1 R2 #7 | Ab12.32 | H R R |
| H2-1-1 R2 #9 | Ab12.33 | A G L |
| H2-1-1 R2 #10 | Ab12.34 | Y G A |
| | | |
| **H2-1-2 WT** | | **I Y Y** |
| H2-1-2 R2 #1 | Ab12.35 | T G P |
| H2-1-2 R2 #2 | Ab12.36 | G G V |
| H2-1-2 R2 #6 | Ab12.37 | V A I |
| H2-1-2 R2 #7 | Ab12.38 | A Y G |
| H2-1-2 R2 #8 | Ab12.39 | V G M |
| H2-1-2 R2 #9 | Ab12.40 | V G A |
| | | |
| **H2-1-2 WT** | | **I Y Y** |
| H2-1-2 R2 #11 | Ab12.41 | Q G H |
| H2-1-2 R2 #12 | Ab12.42 | V W G |
| H2-1-2 R2 #13 | Ab12.43 | G T S |
| H2-1-2 R2 #14,15 | Ab12.44 | V E S |
| H2-1-2 R2 #16 | Ab12.45 | V H M |
| H2-1-2 R2 #17 | Ab12.46 | V G L |
| H2-1-2 R2 #18 | Ab12.47 , | C A G |
| H2-1-2 R2 #19 | Ab12.48 | Y G G |
| H2-1-2 R2 #20 (#5 from 1c/2c) | Ab12.49 | T T E |
| | | |
| **H2-1-3 WT** | | **Y Y S** |
| H2-1-3 R2 #1 | Ab12.1 | A S G |
| H2-1-3 R2 #2 | Ab12.2 | G A G |
| H2-1-3 R2 #3 | Ab12.3 | G N G |
| H2-1-3 R2 #4 | Ab12.4 | A G G |
| H2-1-3 R2 #5 | Ab12.5 | G G H |
| H2-1-3 R2 #6 | Ab12.6 | G G E |
| H2-1-3 R2 #7,8,9 | Ab12.7 | G G G |
| H2-1-3 R2 #10 | Ab12.8 | M G G |
| | | |
| **H2-1-3 WT** | | **Y Y S** |
| H2-1-3 R2 #11 | Ab12.55 | A G E |
| H2-1-3 R2 #12,13,24,25,27-31) | Ab12.56 | A G T |
| H2-1-3 R2 #14,15 | Ab12.107 | G V G |
| H2-1-3 R2 #16 | Ab12.108 | A D E |
| H2-1-3 R2 #17 | Ab12.109 | E V G |
| H2-1-3 R2 #18 | Ab12.110 | A D G |
| H2-1-3 R2 #19 | Ab12.111 | A G G |
| H2-1-3 R2 #20 | Ab12.112 | G V S |
| H2-1-3 R2 #21 | Ab12.113 | G V T |
| H2-1-3 R2 #22 | Ab12.114 | E G G |
| H2-1-3 R2 #23 | Ab12.115 | G E E |
| H2-1-3 R2 #26 | Ab12.116 | T E R |
| | | |
| **H2-4-1 WT** | | **Y S G** |
| H2-4-1 R2 #1 | Ab12.64 | P F S |
| H2-4-1 R2 #2 | Ab12.65 | S P V |
| H2-4-1 R2 #3 | Ab12.66 | P P F |
| H2-4-1 R2 #5 | Ab12.67 | P G V |
| H2-4-1 R2 #6 | Ab12.68 | S P I |
| H2-4-1 R2 #7 | Ab12.69 | P F T |
| H2-4-1 R2 #8,9 | Ab12.70 | S P S |
| H2-4-1 R2 #10 | Ab12.71 | P S I |
| H2-4-1 WT #4 | Ab12 | Y S G |

To determine which clones from the affinity maturation would be converted into an IgG2/K format, outputs from each library were analyzed and considered for the following parameters: frequency of isolation, consensus sequence change in the CDR, and overall fluorescent shift of bulk outputs and individual yeast clones. Those clones appearing at a higher frequency, containing a representative consensus change in CDR sequence and having the highest overall FL2 signal in off-rate and equilibrium binding analyses were chosen for conversion.

### Example 6: Cloning and Expression of Yeast Display-Derived Antibodies

Selected scFvs were converted into IgG2/K antibodies by PCR amplification of the variable domains, followed by ligation of these domains to an intact IgG2 constant region or K region present in the vector pBOS (Mizushima and Nagata, Nucleic Acids Research, Vol 18, pg 5322, 1990). pBOS plasmids encoding both heavy and light chain regions were transfected transiently into COS cells and resulting supernatants from cell cultures were purified over a protein A sepharose column. Purified antibodies were dialyzed into phosphate buffered saline (PBS) and quantitated by optical density 280 (O.D.₂₈₀) spectrophotometric reading. Each antibody was subjected to affinity measurements by BIAcore and used as a test article in UT-7/Epo and F36E cell proliferation assays.

### Example 7: BIAcore Analysis of Yeast Display-Derived Antibodies

BIAcore analyses were performed on a BIAcore 2000 utilizing the BIAcontrol software version 3.1.0 and on a BIAcore 3000 utilizing the BIAcontrol software version 4.0.1. (BIAcore, Uppsala, Sweden) using EposR as the test antigen. Table 2 highlights the affinity parameters of each mutated Ab12 clone compared to Ab12.

**Table 2**

| Name | K*ₒₙ* (1/M x s) | K*_{off}* (1/s) | K*_{d}* (nM) |
|---|---|---|---|
| Ab12 | 1.4 x 10⁵ | 1.3 x 10⁻³ | 11 |
| Ab12.6 | 1.5 x 10⁵ | 4.8 x 10⁻³ | 32 |
| Ab12.56 | 9.4 x 10⁴ | 1.9 x 10⁻³ | 20 |
| Ab12.17 | 1.4 x 10⁵ | 4.5 x 10⁻⁵ | 0.33 |
| Ab12.25 | 6.5 x 10⁴ | 7 x 10⁻⁵ | 1 |
| Ab12.61 | 8.5 x 10⁴ | 9.0 x 10⁻⁵ | 1 |
| Ab12.70 | 1.6 x 10⁵ | 9.9 x 10⁻⁴ | 6 |
| Ab12.76 | 2.1 x 10⁵ | 9.9 x 10⁻⁵ | 0.48 |

As Table 2 shows, Ab12.6 and Ab12.56 showed faster off-rates and higher K*_{d}* values relative to Ab12.

### Example 8: Generation of Sub-variants of Ab12.6

To determine the contribution of the amino acid substitutions present in the Ab12.6 sequence, sub-variants were synthesized using Ab12.6 IgG2/K DNA and suitable PCR primers designed to create substitutions where appropriate. Sub-variants also were subjected to BIAcore analyses as described above. Table 3 highlights the affinity parameters of each subvariant clone.

**Table 3**

| Name | K*ₒₙ* (1/M x s) | K*_{off}* (1/s) | K*_{d}* (nM) |
|---|---|---|---|
| Ab2.118 | 2.5 x 10⁵ | 5.5 x 10⁻³ | 22 |
| Ab12.119 | 2.1 x 10⁵ | 4.4 x 10⁻³ | 21 |
| Ab12.120 | 2.7 x 10⁵ | 2 x 10⁻³ | 7 |
| Ab12.121 | 2.1 x 10⁵ | 6.3 x 10⁻³ | 31 |
| Ab12.122 | 2.2 x 10⁵ | 4.9 x 10⁻³ | 23 |
| Ab12.123 | 1.3 x 10⁵ | 3.3 x 10⁻³ | 25 |

### Example 9: Antibody Dependent Human Cell Proliferation Assay

Ab12 Ab12.6 and Ab12.6-related variants were tested in established *in vitro* cell proliferation assays. Stock cultures of the human erythroleukemic cell lines, UT-7/Epo, or F36E cells were maintained in DMEM or RPMI 1640 media respectively with 10% fetal bovine serum and 1 unit per mL of recombinant human erythropoietin. Prior to assays, cells were cultured overnight at a density of 4.0 to 5.0 x 10⁵ cells per mL in growth medium without Epo. Cells were recovered, washed and resuspended at a density of 1.0 x 10⁶ cells per mL in assay medium (RPMI 1640 or DMEM + 10% FBS) and 50 uL of cells added to wells of a 96 well microtiter plate. 50 uL of each of Ab or Epo standard (recombinant human Epo (rHuEpo)) in assay medium were added to wells at final concentrations ranging from 25nm to 0.098 nm and the plates were incubated in a humidified incubator at 37°C with a 5% CO₂ atmosphere. After 72 hours, 20 µL of Promega Cell Titer 96 Aqueous® reagent (as prepared per manufacturer's instructions, Madison, Wisconin) was added to all wells. Plates were incubated at 37°C with a 5% CO₂ atmosphere for 4 hours and the optical density at 490 nm was determined in a Spectra Max 190 plate reader.

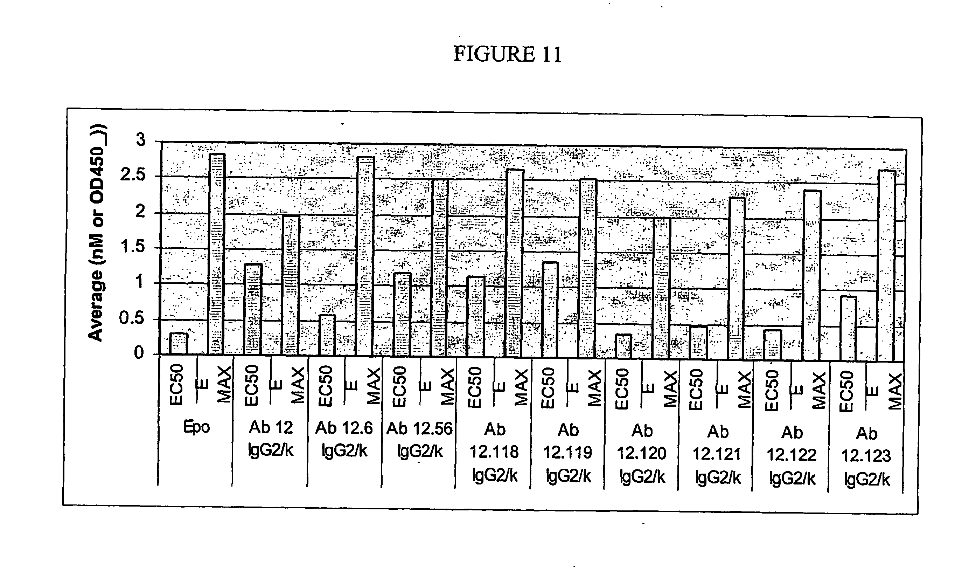

EC₅₀ and Emax values (shown in Table 4 below) were determined from graphs generated from the spectrophotometric data. Higher affinity antibodies (Ab12.17, Ab12.25, Ab12.61 and Ab12.76) produced bell-shaped curves from which EC₅₀ and/or Emax data could not be obtained. In contrast, curves generated from the lower affinity antibodies (shown in Table 4) produced sigmoidal curves (as does the native ligand Epo). Furthermore, as Table 4 and Figure 11 show, Ab12.6 and Ab12.6-related variants (with the exception of Ab12.119) unexpectedly stimulated cell proliferation to a greater extent than Ab12.

**Table 4**

| Test Material | EC₅₀ | Emax |
|---|---|---|
| Epo | 0.297 | 2.82 |
| Ab12 | 1.29 | 1.98 |
| Ab12.6 | 0.58 | 2.81 |
| AB12.56 | 1.17 | 2.512 |
| Ab12.118 | 1.13 | 2.65 |
| Ab12.119 | 1.34 | 2.53 |
| Ab12.120 | 0.34 | 2 |
| Ab12.121 | 0.465 | 2.3 |
| Ab12.122 | 0.42 | 2.4 |
| Ab12.123 | 0.91 | 2.7 |

### Example 10: Construction of mEpoR -/-, hEopR + transgenic mice

Transgenic mice that produced only human EpoR (hEpoR+, single allele) and no endogenous mouse EpoR (mEpoR -/-, double allele mutation) were generated as described in Liu, C. et al., Jounal of Biological Chemistry, 272:32395 (1997) and Yu, X., et al., Blood, 98(2):475 (2001). Breeding colonies were established to generate mice for *in vivo* studies of erythropoiesis.

### Example 11: Human Bone Marrow CFU-E Assay

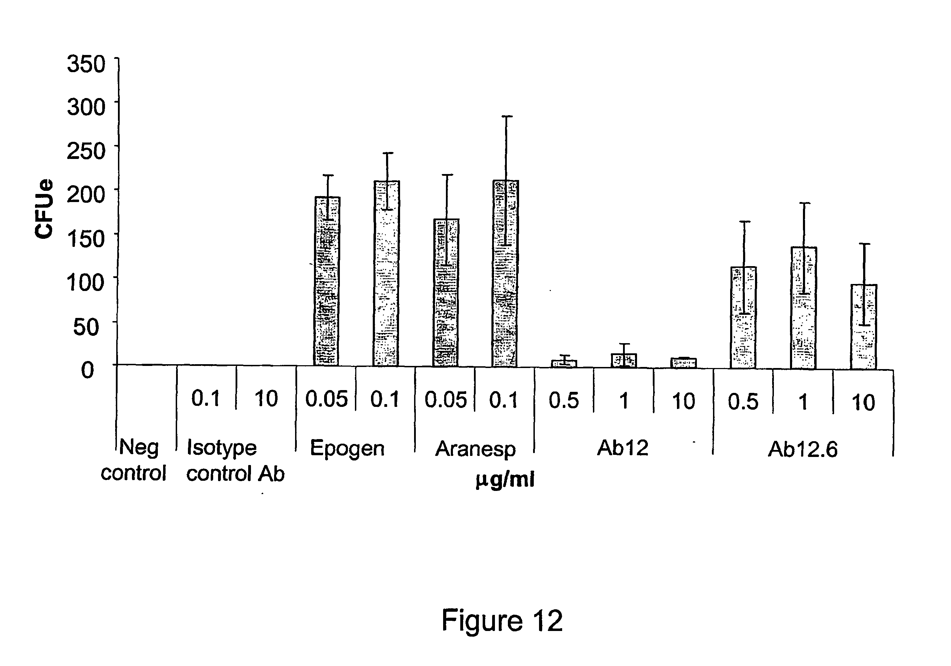

Fresh human bone marrow obtained from Cambrex Bio Science Walkersville, Inc. (Walkersville, MD) were cleared of red blood cells by methods well known in the art and resuspended at 2.5x10⁶ cells/mL in IMDM-2%FBS. Cells (0.1 mL) were added to 17 x 100 mm culture tubes (VWR, West Chester, PA) containing 2.4 mL Methocult (StemCell Technologies, Vancouver, Canada), 0.6 mL ofIMDM-2% FBS , 0.066 mL stem cell growth factor (Sigma, St. Louis, Missouri, 1µg/mL), and Epogen™ (Dik Drug Co., Chicago, IL), Aranesp™ (Dik Drug Co.), Ab12, Ab12.6 or isotype control Ab at the concentrations indicated. After mixing, 1.1 mL of the Methocult suspension was added to a 35 mm non tissue culture treated sterile petri dish and incubated at 37°C, 5% CO₂ for 2 weeks. Colonies, identified microscopically, were red in color. The results in FIG. 12 indicate that Ab 12.6 was more effective than Ab12 in supporting the formation of human CFU-E colonies.

### Example 12: Transgenic Mouse Bone Marrow CFU-E Assay

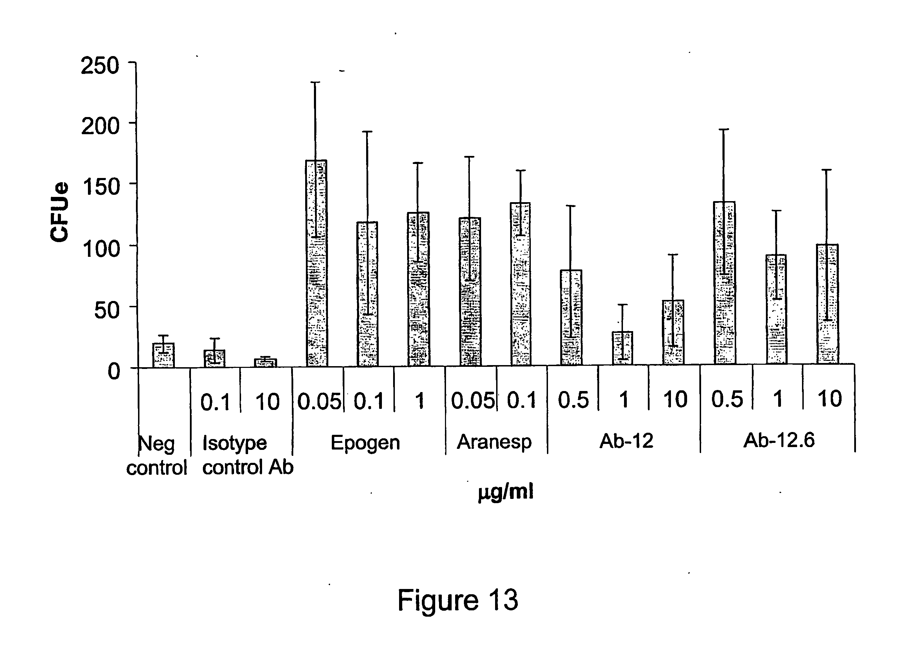

Fresh harvested bone marrow collected from femurs of mEpoR-/-, hEpoR+ transgenic mice were cleared of red blood cells by methods well known in the art and resuspended at 2x10⁶ cells/mL in IMDM-2%FBS. Cells (0.1 mL) were added to 17 x 100 mm culture tubes (VWR, West Chester, PA) containing 3.0 ml Methocult (StemCell Technologies, Vancouver, Canada), 0.165 mL stem cell growth factor (Sigma, St. Louis, Missouri, 1µg/mL), and Epogen™ (Dik Drug Co., Chicago, IL), Aranesp™ (Dik Drug Co.), Ab12, Ab12.6, or isotype control Ab at the concentrations indicated. After mixing, 1.1 mL of the Methocult suspension was added to a 35 mm non tissue culture treated sterile petri dish and incubated at 37°C, 5% CO₂ for 2 weeks. Colonies stained with benzidine (Reference Fibach, E., 1998 Hemoglobin, 22:5-6, 445-458) were identified microscopically. The results in FIG. 13 indicate that Ab12.6 was more effective than Ab12 in supporting the formation of transgenic mouse CFU-E colonies similar to the results observed in the human CFU-E assay (see FIG. 12 above).

### Example 13: Effect of Administration of Ab-12.6 on hematocrit change in mEpoR-/-, hEpoR+ transgenic mouse

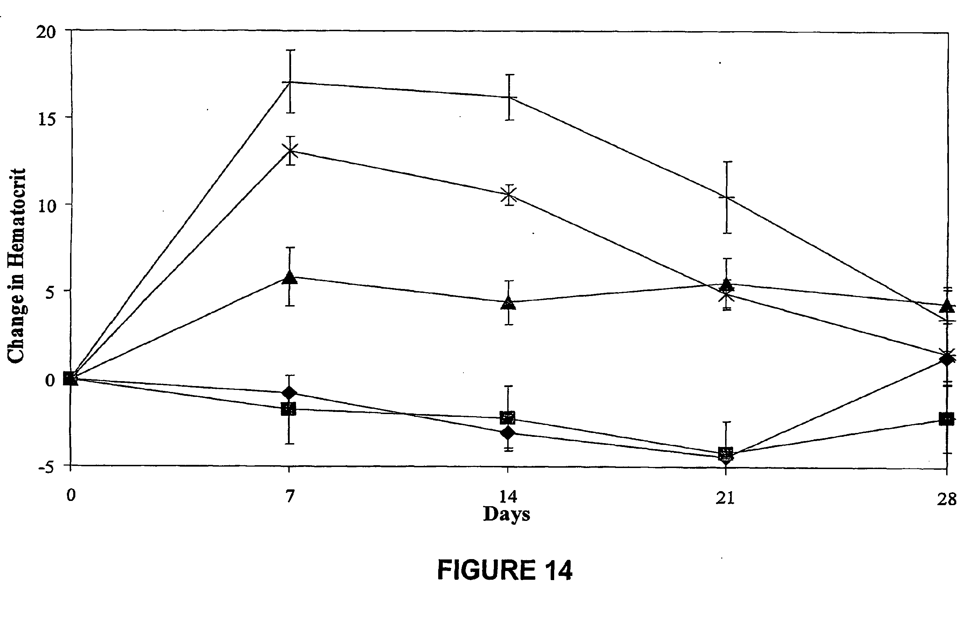

Experiments were performed to determine the effect of a single dose of Ab12.6 on erythropoiesis relative to Aranesp™ (Amgen, Thousand Oaks, CA), a longer acting variant of Epogen. Transgenic mice (mEpoR-/-, hEpoR+ as described in Example 10) were injected subcutaneously once with Ab-12 , Ab12.6 or an isotype control Ab at 0.8 mg/kg in 0.2 mL vehicle (phosphate buffered saline [PBS] containing 0.2% bovine serum albumin [BSA]). Control animals were injected the same way with Aranesp™ at 3 µg/kg only a second Aranesp™ dose also was administered on day 14 (the standard of care Aranesp™ dosing regimen is ~ 3 µg/kg administered biweekly). Sample bleeds were taken on day 0, 7,14, 21 and 28 for determining hematocrits by methods well known in the art. As shown in FIG. 14, compared to Ab12, Ab12.6 had improved potency in elevating and maintaining hematocrit levels over a 28 day time period. Ab12.6 at 0.8 mg/kg also caused a faster rate of rise of hematocrit measured on day 7 than a single dose Aranesp™ administered at 3 µg/kg. In addition a single dose of Ab12.6 was at least as efficacious in elevating the hematocrit on day 28 as Aranesp™ dosed twice on day 0 and day 14.

### Example 14: Generation of linker library

Degenerate oligonucleotide linkers, 45 nucleotides in length were generated according to the following design: 5' GGA NHS NHS NHS NHS NHS NHS NHS NHS NHS NHS NHS NHS NHS AGT 3' (SEQ ID NO:28) and 5' GGA VNS VNS VNS VNS VNS VNS VNS VNS VNS VNS VNS VNS VNS AGT 3' (SEQ ID NO:29) wherein N is A or G or C or T; V is A or C or G; H is A or C or T; and S is C or G.

In the first linker sequence, the use of the NHS codon prevents the creation of GGC and GGG, the two possible codons for glycine in this biased codon selection. In addition, the NHS codon prevents creation of TGC (only possible codon for cysteine), and TGG (only possible codon for tryptophan), CGC, CGG, and AGG (all possible codons for arginine), and AGC (one of three possible serine codons). In the lower linker sequence, the use of the VNS codon limits the creation of TCC and TCG, two of three possible serine codons. In addition, the VNS codon prevents creation of TTC (only possible codon for phenylalanine), TAC (only possible codon for tyrosine), TGC (only possible codon for cysteine), TGG (only possible codon for tryptophan), TAG (only possible stop codon), and TTG (one of three possibly codons for leucine). These linker sequences were synthesized as part of a longer synthetic oligonucleotide which also contained complementary elements to a portion of a control scFv DNA sequence LT28-8A having a (G₄S)₃ linker sequence. LT28-SA was generated using standard molecular biological techniques by replacing the CDR3 sequence ofLT28 Ala-Ala-Trp-Asp-Asp-Ser-Leu-Ser-Gly-Pro-Val (described in WO 01/58956, published August 16, 2001) with Ala-Ala-Gly-Asp-Asp-Phe-Leu-Val-Ser-Met-Leu. Linker sequence (G₄S)₃ is described in US Patent Nos. 5,258,498 and 5,482,858.

The extended linker library oligonucleotides were incorporated into the LT-28-8A scFv by PCR.

NHS- and VNS-linker PCR products were generated, purified and mixed with restriction-digested yeast-display plasmid (pYD-1) containing homologous regions of complementary DNA sequence present in both the 5' and 3' termini of NHS- and VNS-linker PCR products. PCR generated products encoding the entire LT28-8A scFv (with an NHS or VNS-encoded linker) were inserted by homologous recombination into the galactose-inducible pYD-1 vector such that they were in-frame. Homologous recombinants were selected by subsequent growth in tryptophan- and uracil-minus media. Titers of the resulting NHS- and VNS-linker libraries were assessed by colony counts and the libraries were prepared for analysis by a fluorescense-activated cell sorter (FACS).

### Example 15: Analysis of Library

Dot plots of NHS- and VNS-linker LT28-8A scFv libraries from Example 14 were compared with those of LT-28-8A scFv when induced yeast cells from all groups were incubated with V5-FTTC monoclonal antibody (Invitrogen, Carlsbad, CA). The VS epitope tag was encoded within the scFv and was at the 3' end of the polypeptide, and as a result the presence of this epitope indicated that the scFv was fully translated and the signal generated by antibody binding was representative of expression levels of the scFv on the surface of yeast. Percent of cells staining positive for FITC: LT-28-8A was 58%; NHS-library was 31%; and VNS-library was 47%.

FACS analysis of cells from the three test groups were compared following the addition of biotinylated IL-18, prepared as described in WO 01/58956, and streptavidin R-phycoerythin (RPE) (Jackson ImmunoResearch, West Grove, PA) and V5-FITC. The fluorescence of RPE represents the binding of antigen to the scFvs on the surface of yeast, and, in conjunction with the presence of the V5 epitope, a dual-color signal is generated by clones that express full-length scFv and bind antigen. A concentration of 30 nM biotinylated IL-18 was chosen for this analysis because the LT-28-8A scFv had a K_{D} of 30 nM on the surface of yeast. Percent of cells staining positive for both FITC and RPE: LT-28-8A was 55%; NHS-library was 25%; and VNS-library was 36%.

Cells from the NHS- or VNS- LT28-8A scFv libraries that demonstrated fluorescence identical to control were individually gated and collected using a cell sorter. These collected populations (termed the "outputs") were amplified in liquid culture and aliqouts of culture were plated on to solid media to isolate individual colonies. DNA was extracted from individual colonies and the linker nucleotide sequence was determined by DNA sequencing.

### Example 16: Comparison of scFvs containing_Variable Linker Sequences

To determine if linkers containing variable amino acids had any effect on the behavior of scFvs *in vitro* or *in vivo*, 11 random NHS-R1 output scFv clones from Example 15, containing linkers with only one glycine and serine, were selected and tested in a series of assays.

### Example 17: K_{d} measurement on the surface of yeast

The dissociation constants (K*_{d}*) of 11 NHS-R1 output scFv clones and LT-28-8A scFv (with the (G₄S)₃ linker) were measured in a seven-point titration analysis. These included NHS-R1 output scFv clones: 13, 19, 22, 23, 30, 33, 34, 38, 40, 41, and 44. Binding of antigen was assayed as described in Example 14. All NHS-R1 output scFv clones and control scFv showed K*_{d}*s of about 22-26 nM.

### Example 18: Expression and purification of soluble scFv in bacteria

Expression, *in vivo*, of 10 NHS-R1 output clones (10, 13, 19, 30, 33, 34, 38, 40, 41, 44) and LT-28-8A scFv were analyzed following the construction of expression constructs encoding the scFvs. All 10 LT28-8A scFv sequences were ligated into the pUC19/pCANTAB (US Patent No. 5,872,215) inducible expression vector and transformed into TG-1 cells. Following growth under restricted expression, scFv induction was initiated by addition of 1mM IPTG and soluble scFv was affinity purified from periplasmic preparations of induced TG-1 cells. Clones 13, 19 and 30 grew very poorly and were not induced. Purified scFv was assayed for protein concentration by BCA assay:

**Table 5**

| Sample | Concentration (µg/mL) | Sample | Concentration (µg/mL) |
|---|---|---|---|
| NHS-R1-10 | 41 | NHS-R1-40 | 4156 |
| NHS-R1-33 | 826 | NHS-R1-41 | 607 |
| NHS-R1-34 | 1600 | NHS-R1-44 | 55 |
| NHS-R1-38 | 619 | LT-28-8A | 516 |

### Example 19: Activity of soluble scFv in a bioassay

The soluble scFvs produced by LT-28-8A and NHS-R1 output scFv clones 33, 34, 38, 40, 41, and 44 were tested in a neutralization bioassay as described in WO 01/58956. All scFv preparations showed IC₅₀ values of about 1x10⁻⁷ to 2x10⁻⁷ M. Linker sequence 33 is SEQ ID NO:27; Linker sequence 34 is SEQ ID NO:4; Linker sequence 40 is SEQ ID NO:3; Linker sequence 41 is SEQ ID NO:2.

### Example 20: Ab12.6 recognizes a conformational-dependent epitope

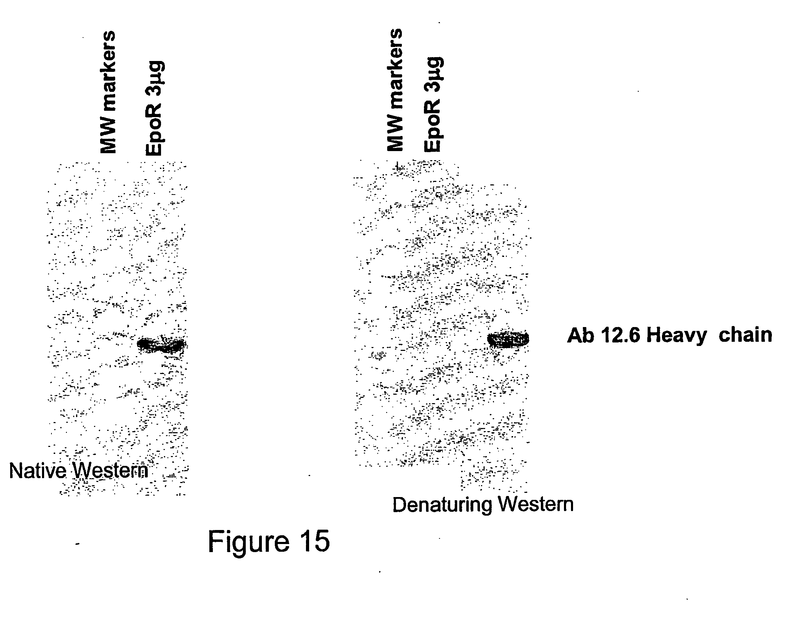

Recombinant-expressed EpoR extracellular domain was produced through CHO cell expression and purified to homogeneity. Three micrograms of EpoR extracelluar domain per lane were electrophoresed on 4-20% poly-acrylamide gels under either denaturing conditions (in SDS buffer) or native conditions (no SDS buffer). For Western blot analysis, gels were transferred to PVDF membranes, blocked with 5% dry milk and incubated with Ab 12.6 (10 µg/ml) for 1-2 h at room temperature. Membranes were washed four times with PBS/Tween, incubated with HRP conjugated goat anti-human antibody (1:2500) and developed with 4-chloro-1-naphthol as substrate. As FIG. 15 shows, AB12.6 interacts with recombinant EpoR extracellular domain only under native, and not under denaturing conditions, indicating that Ab12.6 recognizes a conformational-dependent epitope.

### Example 21: Monomeric Ab12.6 Fab activates EpoR

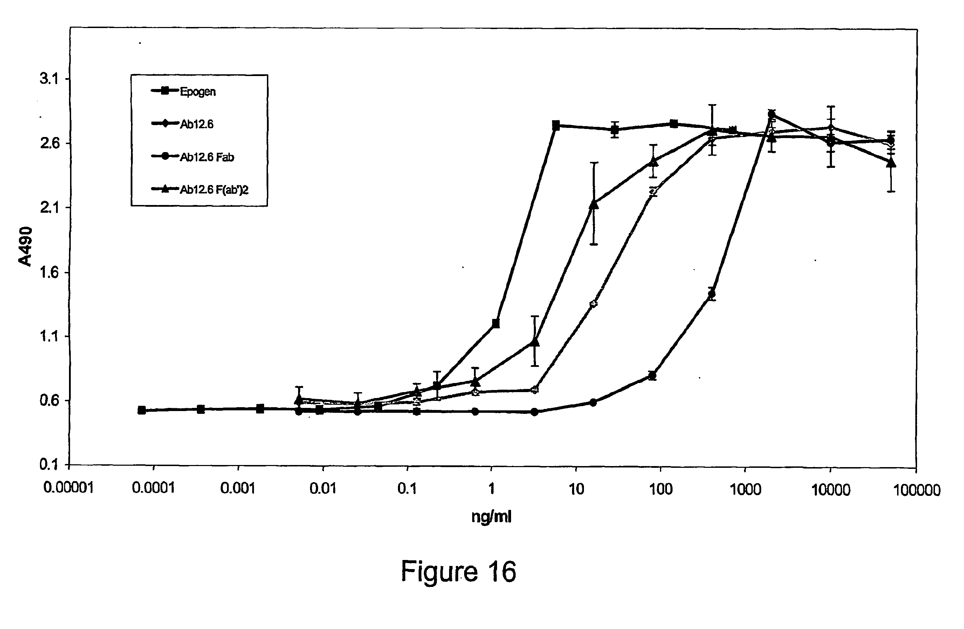

Monmeric Fab and bivalent F(ab')₂ fragments of Ab12.6 were prepared and purified using standard papain and pepsin digestion conditions (Pierce ImmunoPure Fab and F(ab')₂ Preparation Kits; Pierce, Rockford IL). Stock cultures of the human erythroleukemic cell line, F36E cells were maintained in RPMI 1640 media with 10% fetal bovine serum and 1 unit per mL of recombinant human erythropoietin. Prior to assays, cells were cultured overnight at a density of 4.0 to 5.0 x 10⁵ cells per mL in growth medium without EPO. Cells were recovered, washed and resuspended at a density of 1.0 x 10⁶ cells per mL in assay medium (RPMI 1640 + 10% FBS) and 50 uL of cells added to wells of a 96 well microtiter plate. Fifty uL of each of Ab12.6, Ab12.6 Fab, Ab12.6 F(ab')₂ or EPO standards (recombinant human EPO (rHuEPO)) in assay medium were added to wells and the plates were incubated in a humidified incubator at 37°C with a 5% CO₂ atmosphere. After 72 hours, 20 µL of Promega Cell Titer 96 Aqueous® reagent (as prepared per manufacturer's instructions, Madison, Wisconin) was added to all wells. Plates were incubated at 37°C with a 5% CO₂ atmosphere for 4 hours and the optical density at 490 nm was determined using a microplate reader (Wallac Victor 1420 Multilabel Counter, Wallac Company, Boston, MA). The results, seen in FIG 16 saw that the monomeric Ab12.6 Fab stimulated proliferation of the F36E cell line.

The present invention is illustrated by way of the foregoing description and examples.

### SEQUENCE LISTING

<110> Abbott Laboratories
   Reilly, Edward B.
   Lacy, Susan E.
   Fung, Emma
   Belk, Johathan P.
   Roguska, Michael
<120> Antibodies To Erythropoietin Receptor And Uses Thereof
<130> 7349W001
<140>
   <141>
<150> 10/821,497
   <151> 2004-04-09
<150> 10/822,306
   <151> 2004-04-12
<160> 29
<170> FastSEQ for Windows Version 4.0
<210> 1
   <211> 15
   <212> PRT
   <213> Artificial Sequence
<220>
   <223> scFv linker
<400> 1
   Gly Gly Gly Gly Ser Gly Gly Gly Gly Ser Gly Gly Gly Gly Ser
   1 5 10 15
<210> 2
   <211> 15
   <212> PRT
   <213> Artificial Sequence
<220>
   <223> scFv linker
<400> 2
   Gly Glu Asn Lys Val Glu Tyr Ala Pro Ala Leu Met Ala Leu Ser
   1 5 10 15
<210> 3
   <211> 15
   <212> PRT
   <213> Artificial Sequence
<220>
   <223> scFv linker
<400> 3
   Gly Pro Ala Lys Glu Leu Thr Pro Leu Lys Glu Ala Lys Val Ser
   1 5 10 15
<210> 4
   <211> 15
   <212> PRT
   <213> Artificial Sequence
<220>
   <223> scFv linker
<400> 4
   Gly His Glu Ala Ala Ala Val Met Gln Val Gln Tyr Pro Ala Ser
   1 5 10 15
<210> 5
   <211> 116
   <212> PRT
   <213> Homo sapiens
<400> 5
   Gln Val Gln Leu Gln Glu Ser Gly Pro Gly Leu Val Lys Pro Ser Glu
    1               5                  10                  15
   Thr Leu Ser Leu Thr Cys Thr Val Ser Gly Gly Ser Ile Ser Ser Tyr
               20                  25                  30
   Tyr Trp Ser Trp Ile Arg Gln Pro Pro Gly Lys Gly Leu Glu Trp Ile
           35                  40                  45
   Gly Tyr Ile Tyr Tyr Ser Gly Ser Thr Asn Tyr Asn Pro Ser Leu Lys
       50                  55                  60
   Ser Arg Val Thr Ile Ser Val Asp Thr Ser Lys Asn Gln Phe Ser Leu
   65                  70                  75                  80
   Lys Leu Ser Ser Val Thr Ala Ala Asp Thr Ala Val Tyr Tyr Cys Ala
                   85                  90                  95
   Arg Glu Arg Leu Gly Ile Gly Asp Tyr Trp Gly Gln Gly Thr Leu Val
               100                 105                 110
   Thr Val Ser Ser
           115
<210> 6
   <211> 116
   <212> PRT
   <213> Homo sapiens
<400> 6
   Gln Val Gln Leu Gln Glu Ser Gly Pro Gly Leu Val Lys Pro Ser Glu
    1               5                  10                  15
   Thr Leu Ser Leu Thr Cys Thr Val Ser Gly Ala Ser Ile Ser Ser Tyr
               20                  25                  30
   Tyr Trp Ser Trp Ile Arg Gln Pro Pro Gly Lys Gly Leu Glu Trp Ile
           35                  40                  45
   Gly Tyr Ile Tyr Tyr Ser Gly Ser Thr Asn Tyr Asn Pro Ser Leu Lys
       50                  55                  60
   Ser Arg Val Thr Ile Ser Val Asp Thr Ser Lys Asn Gln Phe Ser Leu
   65                  70                  75                  80
   Lys Leu Arg Ser Val Thr Ala Ala Asp Thr Ala Val Tyr Tyr Cys Ala
                   85                  90                  95
   Arg Glu Arg Leu Gly Ile Gly Asp Tyr Trp Gly Gln Gly Thr Leu Val
               100                 105                 110
   Thr Val Ser Ser
           115
<210> 7
   <211> 116
   <212> PRT
   <213> Homo sapiens
<400> 7
   Gln Val Gln Leu Gln Glu Ser Gly Pro Gly Leu Val Lys Pro Ser Glu
    1               5                  10                  15
   Thr Leu Ser Leu Thr Cys Thr Val Ser Gly Ala Ser Ile Ser Ser Tyr
               20                  25                  30
   Tyr Trp Ser Trp Ile Arg Gln Pro Pro Gly Lys Gly Leu Glu Trp Ile
           35                  40                  45
   Gly Tyr Ile Gly Gly Glu Gly Ser Thr Asn Tyr Asn Pro Ser Leu Lys
       50                  55                  60
   Ser Arg Val Thr Ile Ser Val Asp Thr Ser Lys Asn Gln Phe Ser Leu
   65                  70                  75                  80
   Lys Leu Arg Ser Val Thr Ala Ala Asp Thr Ala Val Tyr Tyr Cys Ala
                   85                  90                  95
   Arg Glu Arg Leu Gly Ile Gly Asp Tyr Trp Gly Gln Gly Thr Leu Val
               100                 105                 110
   Thr Val Ser Ser
           115
<210> 8
   <211> 116
   <212> PRT
   <213> Homo sapiens
<400> 8
   Gln Val Gln Leu Gln Glu Ser Gly Pro Gly Leu Val Lys Pro Ser Glu
    1               5                  10                  15
   Thr Leu Ser Leu Thr Cys Thr Val Ser Gly Ala Ser Ile Ser Ser Tyr
               20                  25                  30
   Tyr Trp Ser Trp Ile Arg Gln Pro Pro Gly Lys Gly Leu Glu Trp Ile
           35                  40                  45
   Gly Tyr Ile Ala Gly Thr Gly Ser Thr Asn Tyr Asn Pro Ser Leu Lys
       50                  55                  60
   Ser Arg Val Thr Ile Ser Val Asp Thr Ser Lys Asn Gln Phe Ser Leu
   65                  70                  75                  80
   Lys Leu Arg Ser Val Thr Ala Ala Asp Thr Ala Val Tyr Tyr Cys Ala
                   85                  90                  95
   Arg Glu Arg Leu Gly Ile Gly Asp Tyr Trp Gly Gln Gly Thr Leu Val
               100                 105                 110
   Thr Val Ser Ser
           115
<210> 9
   <211> 116
   <212> PRT
   <213> Homo sapiens
<400> 9
   Gln Val Gln Leu Gln Glu Ser Gly Pro Gly Leu Val Lys Pro Ser Glu
   1 5 10 15
   Thr Leu Ser Leu Thr Cys Thr Val Ser Gly Ala Ser Ile Ser Ser Tyr
   20 25 30
   Tyr Trp Ser Trp Ile Arg Gln Pro Pro Gly Lys Gly Leu Glu Trp Ile
   35 40 45
   Gly Tyr Ile Gly Tyr Ser Gly Ser Thr Asn Tyr Asn Pro Ser Leu Lys
   50 55 60
   Ser Arg Val Thr Ile Ser Val Asp Thr Ser Lys Asn Gln Phe Ser Leu
   65 70 75 80
   Lys Leu Arg Ser Val Thr Ala Ala Asp Thr Ala Val Tyr Tyr Cys Ala
   85 90 95
   Arg Glu Arg Leu Gly Ile Gly Asp Tyr Trp Gly Gln Gly Thr Leu Val
   100 105 110
   Thr Val Ser Ser
   115
<210> 10
   <211> 116
   <212> PRT
   <213> Homo sapiens
<400> 10
   Gln Val Gln Leu Gln Glu Ser Gly Pro Gly Leu Val Lys Pro Ser Glu
   1 5 10 15
   Thr Leu Ser Leu Thr Cys Thr Val Ser Gly Ala Ser Ile Ser Ser Tyr
   20 25 30
   Tyr Trp Ser Trp Ile Arg Gln Pro Pro Gly Lys Gly Leu Glu Trp Ile
   35 40 45
   Gly Tyr Ile Tyr Gly Ser Gly Ser Thr Asn Tyr Asn Pro Ser Leu Lys
   50 55 60
   Ser Arg Val Thr Ile Ser Val Asp Thr Ser Lys Asn Gln Phe Ser Leu
   65 70 75 80
   Lys Leu Arg Ser Val Thr Ala Ala Asp Thr Ala Val Tyr Tyr Cys Ala
   85 90 95
   Arg Glu Arg Leu Gly Ile Gly Asp Tyr Trp Gly Gln Gly Thr Leu Val
   100 105 110
   Thr Val Ser Ser
   115
<210> 11
   <211> 116
   <212> PRT
   <213> Homo sapiens
<400> 11
   Gln Val Gln Leu Gln Glu Ser Gly Pro Gly Leu Val Lys Pro Ser Glu
    1               5                  10                  15
   Thr Leu Ser Leu Thr Cys Thr Val Ser Gly Ala Ser Ile Ser Ser Tyr
               20                  25                  30
   Tyr Trp Ser Trp Ile Arg Gln Pro Pro Gly Lys Gly Leu Glu Trp Ile
           35                  40                  45
   Gly Tyr Ile Tyr Tyr Glu Gly Ser Thr Asn Tyr Asn Pro Ser Leu Lys
       50                  55                  60
   Ser Arg Val Thr Ile Ser Val Asp Thr Ser Lys Asn Gln Phe Ser Leu
   65                  70                  75                  80
   Lys Leu Arg Ser Val Thr Ala Ala Asp Thr Ala Val Tyr Tyr Cys Ala
                   85                  90                  95
   Arg Glu Arg Leu Gly Ile Gly Asp Tyr Trp Gly Gln Gly Thr Leu Val
               100                 105                 110
   Thr Val Ser Ser
           115
<210> 12
   <211> 116
   <212> PRT
   <213> Homo sapiens
<400> 12
   Gln Val Gln Leu Gln Glu Ser Gly Pro Gly Leu Val Lys Pro Ser Glu
    1               5                  10                  15
   Thr Leu Ser Leu Thr Cys Thr Val Ser Gly Ala Ser Ile Ser Ser Tyr
               20                  25                  30
   Tyr Trp Ser Trp Ile Arg Gln Pro Pro Gly Lys Gly Leu Glu Trp Ile
           35                  40                  45
   Gly Tyr Ile Gly Gly Ser Gly Ser Thr Asn Tyr Asn Pro Ser Leu Lys
       50                  55                  60
   Ser Arg Val Thr Ile Ser Val Asp Thr Ser Lys Asn Gln Phe Ser Leu
   65                  70                  75                  80
   Lys Leu Arg Ser Val Thr Ala Ala Asp Thr Ala Val Tyr Tyr Cys Ala
                   85                  90                  95
   Arg Glu Arg Leu Gly Ile Gly Asp Tyr Trp Gly Gln Gly Thr Leu Val
               100                 105                 110
   Thr Val Ser Ser
           115
<210> 13
   <211> 116
   <212> PRT
   <213> Homo sapiens
<400> 13
   Gln Val Gln Leu Gln Glu Ser Gly Pro Gly Leu Val Lys Pro Ser Glu
    1               5                  10                  15
   Thr Leu Ser Leu Thr Cys Thr Val Ser Gly Ala Ser Ile Ser Ser Tyr
               20                  25                  30
   Tyr Trp Ser Trp Ile Arg Gln Pro Pro Gly Lys Gly Leu Glu Trp Ile
           35                  40                  45
   Gly Tyr Ile Tyr Gly Glu Gly Ser Thr Asn Tyr Asn Pro Ser Leu Lys
       50                  55                  60
   Ser Arg Val Thr Ile Ser Val Asp Thr Ser Lys Asn Gln Phe Ser Leu
   65                  70                  75                  80
   Lys Leu Arg Ser Val Thr Ala Ala Asp Thr Ala Val Tyr Tyr Cys Ala
                   85                  90                  95
   Arg Glu Arg Leu Gly Ile Gly Asp Tyr Trp Gly Gln Gly Thr Leu Val
               100                 105                 110
   Thr Val Ser Ser
           115
<210> 14
   <211> 116
   <212> PRT
   <213> Homo sapiens
<400> 14
   Gln Val Gln Leu Gln Glu Ser Gly Pro Gly Leu Val Lys Pro Ser Glu
    1               5                  10                  15
   Thr Leu Ser Leu Thr Cys Thr Val Ser Gly Ala Ser Ile Ser Ser Tyr
               20                  25                  30
   Tyr Trp Ser Trp Ile Arg Gln Pro Pro Gly Lys Gly Leu Glu Trp Ile
           35                  40                  45
   Gly Tyr Ile Gly Tyr Glu Gly Ser Thr Asn Tyr Asn Pro Ser Leu Lys
       50                  55                  60
   Ser Arg Val Thr Ile Ser Val Asp Thr Ser Lys Asn Gln Phe Ser Leu
   65                  70                  75                  80
   Lys Leu Arg Ser Val Thr Ala Ala Asp Thr Ala Val Tyr Tyr Cys Ala
                   85                  90                  95
   Arg Glu Arg Leu Gly Ile Gly Asp Tyr Trp Gly Gln Gly Thr Leu Val
               100                 105                 110
   Thr Val Ser Ser
           115
<210> 15
   <211> 116
   <212> PRT
   <213> Artificial Sequence
<220>
   <221> VARIANT
   <222> 52, 53, 54
<223> Xaa = Any Amino Acid
<400> 15
   Gln Val Gln Leu Gln Glu Ser Gly Pro Gly Leu Val Lys Pro Ser Glu
    1               5                  10                  15
   Thr Leu Ser Leu Thr Cys Thr Val Ser Gly Ala Ser Ile Ser Ser Tyr
               20                  25                  30
   Tyr Trp Ser Trp Ile Arg Gln Pro Pro Gly Lys Gly Leu Glu Trp Ile
           35                  40                  45
   Gly Tyr Ile Xaa Xaa Xaa Gly Ser Thr Asn Tyr Asn Pro Ser Leu Lys
       50                  55                  60
   Ser Arg Val Thr Ile Ser Val Asp Thr Ser Lys Asn Gln Phe Ser Leu
   65                  70                  75                  80
   Lys Leu Arg Ser Val Thr Ala Ala Asp Thr Ala Val Tyr Tyr Cys Ala
                   85                  90                  95
   Arg Glu Arg Leu Gly Ile Gly Asp Tyr Trp Gly Gln Gly Thr Leu Val
               100                 105                 110
   Thr Val Ser Ser
           115
<210> 16
   <211> 107
   <212> PRT
   <213> Homo sapiens
<400> 16
   Asp Ile Gln Met Thr Gln Ser Pro Ser Ser Leu Ser Ala Ser Val Gly
    1               5                  10                  15
   Asp Arg Val Thr Ile Thr Cys Arg Ala Ser Gln Gly Ile Arg Asn Asp
               20                  25                  30
   Leu Gly Trp Tyr Gln Gln Lys Pro Gly Lys Ala Pro Lys Arg Leu Ile
           35                  40                  45
   Tyr Ala Ala Ser Ser Leu Gln Ser Gly Val Pro Ser Arg Phe Ser Gly
       50                  55                  60
   Ser Gly Ser Gly Thr Glu Phe Thr Leu Thr Ile Ser Ser Leu Gln Pro
   65                  70                  75                  80
   Glu Asp Phe Ala Thr Tyr Tyr Cys Leu Gln His Asn Ser Tyr Pro Pro
                   85                  90                  95
   Thr Phe Gly Gln Gly Thr Lys Val Glu Ile Lys
               100                 105
<210> 17
   <211> 107
   <212> PRT
   <213> Homo sapiens
<400> 17
   Asp Ile Gln Leu Thr Gln Ser Pro Ser Ser Leu Ser Ala Ser Val Gly
    1               5                  10                  15
   Asp Arg Val Thr Ile Thr Cys Arg Ala Ser Gln Gly Ile Arg Asn Asp
               20                  25                  30
   Leu Gly Trp Tyr Gln Gln Lys Pro Gly Lys Ala Pro Lys Arg Leu Ile
           35                  40                  45
   Tyr Ala Ala Ser Ser Leu Gln Ser Gly Val Pro Ser Arg Phe Ser Gly
       50                  55                  60
   Ser Gly Ser Gly Thr Glu Phe Thr Leu Thr Ile Ser Ser Leu Gln Pro
   65                  70                  75                  80
   Glu Asp Phe Ala Thr Tyr Tyr Cys Leu Gln His Asn Thr Tyr Pro Pro
                   85                  90                  95
   Thr Phe Gly Gln Gly Thr Lys Val Glu Ile Lys
               100                 105
<210> 18
   <211> 16
   <212> PRT
   <213> Artificial Sequence
<220>
   <221> VARIANT
   <222> 3,4,5
<223> Xaa = Any Amino Acid
<400> 18
   Tyr Ile Xaa Xaa Xaa Gly Ser Thr Asn Tyr Asn Pro Ser Leu Lys Ser
    1               5                  10                  15
<210> 19
   <211> 16
   <212> PRT
   <213> Homo sapiens
<400> 19
   Tyr Ile Gly Gly Glu Gly Ser Thr Asn Tyr Asn Pro Ser Leu Lys Ser
    1               5                  10                  15
<210> 20
   <211> 16
   <212> PRT
   <213> Homo sapiens
<400> 20
   Tyr Ile Ala Gly Thr Gly Ser Thr Asn Tyr Asn Pro Ser Leu Lys Ser
    1               5                  10                  15
<210> 21
   <211> 16
   <212> PRT
   <213> Homo sapiens
<400> 21
   Tyr Ile Gly Tyr Ser Gly Ser Thr Asn Tyr Asn Pro Ser Leu Lys Ser
   1 5 10 15
<210> 22
   <211> 16
   <212> PRT
   <213> Homo sapiens
<400> 22
   Tyr Ile Tyr Gly Ser Gly Ser Thr Asn Tyr Asn Pro Ser Leu Lys Ser
   1 5 10 15
<210> 23
   <211> 16
   <212> PRT
   <213> Homo sapiens
<400> 23
   Tyr Ile Tyr Tyr Glu Gly Ser Thr Asn Tyr Asn Pro Ser Leu Lys Ser
   1 5 10 15
<210> 24
   <211> 16
   <212> PRT
   <213> Homo sapiens
<400> 24
   Tyr Ile Gly Gly Ser Gly Ser Thr Asn Tyr Asn Pro Ser Leu Lys Ser
   1 5 10 15
<210> 25
   <211> 16
   <212> PRT
   <213> Homo sapiens
<400> 25
   Tyr Ile Tyr Gly Glu Gly Ser Thr Asn Tyr Asn Pro Ser Leu Lys Ser
   1 5 10 15
<210> 26
   <211> 16
   <212> PRT
   <213> Homo sapiens
<400> 26
   Tyr Ile Gly Tyr Glu Gly Ser Thr Asn Tyr Asn Pro Ser Leu Lys Ser
   1 5 10 15
<210> 27
   <211> 15
   <212> PRT
   <213> Artificial Sequence
<220>
   <223> Linker sequence
<400> 27
   Gly Phe Lys Asp Ala Leu Lys Gln Pro Met Pro Tyr Ala Thr Ser
   1 5 10 15
<210> 28
   <211> 45
   <212> DNA
   <213> Artificial Sequence
<220>
   <221> variation
   <222> 4 - 19, 21 - 42
<223 > n = A, T, C or G; h = A, C or T; s = C or G
<400> 28
   gganhsnhsn hsnhsnhsnh snhsnhsnhs nhsnhsnhsn hsagt 45
<210> 29
   <211> 45
   <212> DNA
   <213> Artificial Sequence
<220>
   <221> variation
   <222> 4 - 19, 21 - 42
<223> n = A, T, C or G; s = C or G; v = A, C or G
<400> 29
   ggavnsvnsv nsvnsvnsvn svnsvnsvns vnsvnsvnsv nsagt 45

## Claims

1. An isolated antibody, or an antigen-binding portion thereof, that dissociates from human erythropoietin receptor (EpoR) with a K*_{off}* rate constant of greater than 1.3 x 10⁻³ s⁻¹ and that activates an endogenous activity of said human EpoR in a mammal, said antibody or antigen-binding portion thereof comprising a heavy chain variable region (HCVR) and a light chain variable region, wherein the heavy chain variable region comprises an amino acid sequence of Formula I: wherein:
X₁ is independently selected from the group consisting of tyrosine (Y), glycine (G) and alanine (A);
X₂ is independently selected from the group consisting of tyrosine (Y), glycine (G), alanine (A), glutamine (E) and aspartic acid (D); and
X₃ is independently selected from the group consisting of serine (S), glycine (G), glutamine (E) and threonine (T)
with the proviso that X₁ - X₂ - X₃ is other than Y-Y-S, and
the light chain variable region comprises an amino acid sequence of SEQ ID NO: 17.

2. The antibody or antigen-binding portion thereof of claim 1 wherein said K*_{off}* rate constant is selected from the group consisting of 1.4 x 10⁻³ s⁻¹ or greater, 1.9 x 10⁻³ s⁻¹ and 4.8 x 10⁻³ s⁻¹.

3. The antibody or antigen-binding portion thereof of claim 1 wherein said antibody is a monoclonal antibody.

4. The antibody or antigen-binding portion thereof of claim 3 wherein said antibody is an IgG2 isotype.

5. The antibody or antigen-binding portion thereof of claim 1 which is a human antibody.

6. The antibody or antigen-binding portion thereof of claim 1 that binds to human EpoR with a K*_{d}* of 7 nM or greater.

7. The antibody or antigen-binding portion thereof of claim 6 wherein said K*_{d}* is 7 to 32 nM inclusive.

8. The antibody or antigen-binding portion thereof of claim 1, wherein the heavy chain variable region has an amino acid sequence selected from the group consisting of SEQ ID NO: 7, SEQ ID NO: 8, SEQ ID NO: 9, SEQ ID NO: 10, SEQ ID NO: 11, SEQ ID NO: 12, SEQ ID NO: 13 and SEQ ID NO: 14.

9. The antibody or antigen-binding portion thereof according to any one of claims 1 to 8 for activating an endogenous activity of a human erythropoietin receptor for treating anemia, hypoxemia, chronic tissue hypoxia, heart failure, ischemic heart disease, renal failure, neural cell damage or tissue damage, or for promoting wound healing in a mammal, by administration to said mammal of a therapeutically effective amount of said antibody or antigen-binding portion thereof.

10. The antibody or antigen-binding portion thereof according to any of claims 1 to 8 for treating a mammal suffering aplasia, by administration to a mammal in need of treatment of a therapeutically effective amount of said antibody or antigen-binding portion thereof.

11. The antibody or antigen-binding portion thereof according to any of claims 1 to 8 for treating a mammal suffering anemia, by administration to a mammal in need of treatment of a therapeutically effective amount of said antibody or antigen-binding portion thereof.

12. A pharmaceutical composition comprising a therapeutically effective amount of an antibody or antigen-binding portion thereof according to any of claims 1 to 8 and a pharmaceutically acceptable excipient.

13. An isolated or purified polynucleotide sequence which encodes a heavy chain variable region and a light chain variable region, wherein the heavy chain variable region comprises an amino acid sequence of Formula I: wherein:
X₁ is independently selected from the group consisting of tyrosine (Y), glycine (G) and alanine (A);
X₂ is independently selected from the group consisting of tyrosine (Y), glycine (G), alanine (A); glutamine (E) and aspartic acid (D); and
X₃ is independently selected from the group consisting of serine (S), glycine (G), glutamine (E) and threonine (T)
with the proviso that X₁-X₂ -X₃ is other than Y-Y-S, and
the light chain variable region comprises an amino acid sequence of SEQ ID NO: 17.

14. The polynucleotide of claim 13 wherein said polynucleotide encodes a heavy chain variable region selected from the group consisting of SEQ ID NO: 7, SEQ ID NO: 8, SEQ ID NO: 9, SEQ ID NO: 10, SEQ ID NO: 11, SEQ ID NO: 12, SEQ ID NO: 13 and SEQ ID NO: 14.

15. A recombinant expression vector comprising the polynucleotide of claim 13.

16. A host cell comprising the recombinant expression vector of claim 15.

17. The host cell of claim 16 which is a mammalian cell.

18. The host cell of claim 17 selected from the group consisting of a CHO cell, a COS cell, and an HEK-293 cell.

19. The host cell of claim 16 which is yeast cell.

20. The host cell of claim 16 which is a bacterial cell.

21. A polypeptide sequence encoded by said polynucleotide sequence of claim 13.

## Patentansprüche

1. Ein isolierter Antikörper, oder ein Antigen-bindender Abschnitt davon, der von Human-Erythropoietinrezeptor (EpoR) mit einer K*_{off}*-Geschwindigkeitskonstante von mehr als 1,3 x 10⁻³ s⁻¹ dissoziiert und der eine endogene Aktivität des Human-EpoR in einem Säugetier aktiviert, wobei der Antikörper oder der Antigen-bindende Abschnitt davon eine variable Region der schweren Ketten (Heavy Chain Variable Region - HCVR) und eine variable Region der leichten Kette (Light Chain Variable Region) umfassen, worin die variable Region der schweren Ketten eine Aminosäuresequenz mit der Formel I umfasst: worin:
X₁ unabhängig gewählt ist aus der Gruppe bestehend aus Tyrosin (Y), Glycin (G) und Alanin (A),
X₂ unabhängig gewählt ist aus der Gruppe bestehend aus Tyrosin (Y), Glycin (G), Alanin (A), Glutamin (E) und Asparaginsäure (D), und
X₃ unabhängig gewählt ist aus der Gruppe bestehend aus Serin (S), Glycin (G), Glutamin (E) und Threonin (T),
unter der Voraussetzung, dass X₁ - X₂ - X₃ nicht Y-Y-S ist,
und
die variable Region der leichten Kette eine Aminosäuresequenz mit der Sequenzidentifikationsnummer 17 umfasst.

2. Der Antikörper oder Antigen-bindende Abschnitt davon gemäß Anspruch 1, worin die K*_{off}*-Geschwindigkeitskonstante gewählt ist aus der Gruppe bestehend aus 1,4 x 10⁻³ s⁻¹ oder höher, 1,9 x 10⁻³ s⁻¹ und 4,8 x 10⁻³ s⁻¹.

3. Der Antikörper oder Antigen-bindende Abschnitt davon gemäß Anspruch 1, worin der Antikörper ein monoklonaler Antikörper ist.

4. Der Antikörper oder Antigen-bindende Abschnitt davon gemäß Anspruch 3, worin der Antikörper ein IgG2-Isotyp ist.

5. Der Antikörper oder Antigen-bindende Abschnitt davon gemäß Anspruch 1, der ein Human-Antikörper ist.

6. Der Antikörper oder Antigen-bindende Abschnitt davon gemäß Anspruch 1, der sich an Human-EpoR mit einem K*_{d}* von 7 nM oder höher bindet.

7. Der Antikörper oder Antigen-bindende Abschnitt davon gemäß Anspruch 6, worin der K*_{d}* 7 bis einschließlich 32 nM beträgt.

8. Der Antikörper oder Antigen-bindende Abschnitt davon gemäß Anspruch 1, worin die variable Region der schweren Kette eine Aminosäuresequenz hat, die gewählt ist aus der Gruppe bestehend aus SEQ.-ID.-NR. 7, SEQ.-ID.-NR. 8, SEQ.-ID.-NR. 9, SEQ.-ID.-NR. 10, SEQ.-ID.-NR. 11, SEQ.-ID.-NR. 12, SEQ.-ID.-NR. 13 und SEQ.-ID.-NR. 14.

9. Der Antikörper oder Antigen-bindende Abschnitt davon gemäß einem beliebigen der Ansprüche 1 bis 8 zur Aktivierung einer endogenen Aktivität eines Human-Erythropoietin-Rezeptors für die Behandlung von Anämie, Hypoxämie, chronischer Gewebehypoxie, Herzversagen, ischämischer Herzerkrankung, Nierenversagen, Nervenzellschädigung oder Gewebeschädigung, oder zur Förderung der Wundheilung bei einem Säugetier durch Verabreichung einer therapeutisch wirksamen Menge des Antikörpers oder des Antigen-bindenden Abschnitts davon an das Säugetier.

10. Der Antikörper oder Antigen-bindende Abschnitt davon gemäß einem beliebigen der Ansprüche 1 bis 8 zur Behandlung eines Säugetiers, das an Aplasie leidet, durch Verabreichung einer therapeutisch wirksamen Menge des Antikörpers oder Antigen-bindenden Abschnitts davon an ein Säugetier, das Behandlung benötigt.

11. Der Antikörper oder Antigen-bindende Abschnitt davon gemäß einem beliebigen der Ansprüche 1 bis 8 zur Behandlung eines Säugetiers, das an Anämie leidet, durch Verabreichung einer therapeutisch wirksamen Menge des Antikörpers oder Antigen-bindenden Abschnitts davon an ein Säugetier, das Behandlung benötigt.

12. Eine pharmazeutische Zusammensetzung, die eine therapeutisch wirksame Menge eines Antikörpers oder Antigen-bindenden Abschnitts davon gemäß einem beliebigen der Ansprüche 1 bis 8 und ein pharmazeutisch verträgliches Bindemittel umfasst.

13. Eine isolierte oder gereinigte Polynukleotidsequenz, die eine variable Region der schweren Kette und eine variable Region der leichten Kette codiert, worin die variable Region der schweren Kette eine Aminosäuresequenz mit der Formel I umfasst: worin:
X₁ unabhängig gewählt ist aus der Gruppe bestehend aus Tyrosin (Y), Glycin (G) und Alanin (A),
X₂ unabhängig gewählt ist aus der Gruppe bestehend aus Tyrosin (Y), Glycin (G), Alanin (A), Glutamin (E) und Asparaginsäure (D), und
X₃ unabhängig gewählt ist aus der Gruppe bestehend aus Serin (S), Glycin (G), Glutamin (E) und Threonin (T),
unter der Voraussetzung, dass X₁-X₂-X₃ nicht Y-Y-S ist und
die variable Region der leichten Kette eine Aminosäuresequenz mit der Sequenzidentifikationsnummer 17 umfasst.

14. Das Polynukleotid gemäß Anspruch 13, worin das Polynukleotid eine variable Region der schweren Kette codiert, die gewählt ist aus der Gruppe bestehend aus SEQ.-ID.-NR. 7, SEQ.-ID.-NR. 8, SEQ.-ID.-NR. 9, SEQ.-ID.-NR. 10, SEQ.-ID.-NR. 11, SEQ.-ID.-NR. 12, SEQ.-ID.-NR. 13 und SEQ.-ID.-NR. 14.

15. Ein rekombinierter Expressionsvektor, der das Polynukleotid gemäß Anspruch 13 umfasst.

16. Eine Wirtszelle, die den rekombinierten Expressionsvektor gemäß Anspruch 15 umfasst.

17. Die Wirtszelle gemäß Anspruch 16, die eine Säugetierzelle ist.

18. Die Wirtszelle gemäß Anspruch 17, gewählt aus der Gruppe, die aus einer CHO-Zelle, einer COS-Zelle und einer HEK-293-Zelle besteht.

19. Die Wirtszelle gemäß Anspruch 16, die eine Hefezelle ist.

20. Die Wirtszelle gemäß Anspruch 16, die eine bakterielle Zelle ist.

21. Eine Polypeptidsequenz, die von der Polynukleotidsequenz gemäß Anspruch 13 codiert wird.

## Revendications

1. Anticorps isolé, ou partie de liaison d'antigène de celui-ci, qui se dissocie du récepteur d'érythropoïétine (EpoR) humain avec une constante de vitesse K*_{off}* supérieure à 1,3 x 10⁻³ s⁻¹ et qui active une activité endogène dudit EpoR humain chez un mammifère, ledit anticorps ou sa partie de liaison d'antigène comprenant une région variable de chaîne lourde (HCVR) et une région variable de chaîne légère, où la région variable de chaîne lourde comprend une séquence d'aminoacides de formule 1 : où :
X₁ est choisi indépendamment dans le groupe consistant en la tyrosine (Y), la glycine (G) et l'alanine (A) ;
X₂ est choisi indépendamment dans le groupe consistant en la tyrosine (Y), la glycine (G), l'alanine (A), la glutamine (E) et l'acide aspartique (D) ; et
X₃ est choisi indépendamment dans le groupe consistant en la sérine (S), la glycine (G), la glutamine (E) et la thréonine (T)
avec la condition que X₁ - X₂ - X₃ soit différent de Y-Y-S, et
la région variable de chaîne légère comprend une séquence d'aminoacides de SEQ ID NO:17.

2. Anticorps ou partie de liaison d'antigène de celui-ci selon la revendication 1 où ladite constante de vitesse K*_{off}* est choisie dans le groupe consistant en 1,4 x 10⁻³ s⁻¹ ou plus, 1,9 x 10⁻³ s⁻¹ et 4,8 x 10⁻³ s⁻¹.

3. Anticorps ou partie de liaison d'antigène de celui-ci selon la revendication 1 où ledit anticorps est un anticorps monoclonal.

4. Anticorps ou partie de liaison d'antigène de celui-ci selon la revendication 3 où ledit anticorps est un isotype IgG2.

5. Anticorps ou partie de liaison d'antigène de celui-ci selon la revendication 1 qui est un anticorps humain.

6. Anticorps ou partie de liaison d'antigène de celui-ci selon la revendication 1 qui se lie à EpoR humain avec une K*_{d}* de 7 nM ou plus.

7. Anticorps ou partie de liaison d'antigène de celui-ci selon la revendication 6 où ladite K*_{d}* est 7 à 32 nM inclus.

8. Anticorps ou partie de liaison d'antigène de celui-ci selon la revendication 1 où la région variable de chaîne lourde a une séquence d'aminoacides choisie dans le groupe consistant en SEQ ID NO:7, SEQ ID NO:8, SEQ ID NO:9, SEQ ID NO:10, SEQ ID NO:11, SEQ ID NO:12, SEQ ID NO:13 et SEQ ID NO:14.

9. Anticorps ou partie de liaison d'antigène de celui-ci selon l'une quelconque des revendications 1 à 8 pour activer une activité endogène d'un récepteur d'érythropoïétine humain pour traiter l'anémie, l'hypoxémie, l'hypoxie tissulaire chronique, l'insuffisante cardiaque, la cardiopathie ischémique, l'insuffisance rénale, les lésions des cellules nerveuses ou les lésions des tissus nerveux, ou pour favoriser la cicatrisation des plaies chez un mammifère, par administration audit mammifère d'une quantité thérapeutiquement efficace dudit anticorps ou de sa partie de liaison d'antigène.

10. Anticorps ou partie de liaison d'antigène de celui-ci selon l'une quelconque des revendications 1 à 8 pour traiter un animal souffrant d'aplasie, par administration à un mammifère nécessitant un traitement d'une quantité thérapeutiquement efficace dudit anticorps ou de sa partie de liaison d'antigène.

11. Anticorps ou partie de liaison d'antigène de celui-ci selon l'une quelconque des revendications 1 à 8 pour traiter un animal souffrant d'anémie, par administration à un mammifère nécessitant un traitement d'une quantité thérapeutiquement efficace dudit anticorps ou de sa partie de liaison d'antigène.

12. Composition pharmaceutique comprenant une quantité thérapeutiquement efficace d'un anticorps ou de sa partie de liaison d'antigène selon l'une quelconque des revendications 1 à 8 et un excipient pharmaceutiquement acceptable.

13. Séquence polynucléotidique isolée ou purifiée qui code une région variable de chaîne lourde et une région variable de chaîne légère, où la région variable de chaîne lourde comprend une séquence d'aminoacides de formule I : où :
X₁ est choisi indépendamment dans le groupe consistant en la tyrosine (Y), la glycine (G) et l'alanine (A) ;
X₂ est choisi indépendamment dans le groupe consistant en la tyrosine (Y), la glycine (G), l'alanine (A), la glutamine (E) et l'acide aspartique (D) ; et
X₃ est choisi indépendamment dans le groupe consistant en la sérine (S), la glycine (G), la glutamine (E) et la thréonine (T)
avec la condition que X₁ - X₂ - X₃ soit différent de Y-Y-S, et
la région variable de chaîne légère comprend une séquence d'aminoacides de SEQ ID NO:17.

14. Polynucléotide selon la revendication 13 où ledit polynucléotide code une région variable de chaîne lourde choisie dans le groupe consistant en SEQ ID NO:7, SEQ ID NO:8, SEQ ID NO:9, SEQ ID NO:10, SEQ ID NO:11, SEQ ID NO:12, SEQ ID NO:13 et SEQ ID NO:14.

15. Vecteur d'expression recombinant comprenant le polynucléotide selon la revendication 13.

16. Cellule hôte comprenant le vecteur d'expression recombinant selon la revendication 15.

17. Cellule hôte selon la revendication 16 qui est une cellule de mammifère.

18. Cellule hôte selon la revendication 17 choisie dans le groupe consistant en une cellule CHO, une cellule COS et une cellule HEK-293.

19. Cellule hôte selon la revendication 16 qui est une cellule de levure.

20. Cellule hôte selon la revendication 16 qui est une cellule bactérienne.

21. Séquence polypeptidique codée par ladite séquence polynucléotidique selon la revendication 13.
